# EUROPEAN PATENT SPECIFICATION

(11) **EP 3 768 276 B1**
(45) Date of publication and mention of the grant of the patent: **31.12.2025**
(21) Application number: 19711380.6
(22) Date of filing: 22.03.2019
(51) Int. Cl.: A61K 31/704, A61P 17/02, A61K 9/06, A61K 9/00

(54) **MEANS AND METHODS FOR WOUND HEALING**
MITTEL UND VERFAHREN ZUR WUNDHEILUNG
MOYENS ET PROCÉDÉS DE CICATRISATION DE PLAIE

(30) Priority: 22.03.2018 GB 201804562; 26.03.2018 LU 100740
(43) Date of publication of application: 27.01.2021
(73) Proprietor: VIB VZW, 9052 Gent (BE); Universiteit Gent, 9000 Gent (BE); Katholieke Universiteit Leuven, K.U.Leuven R&D, 3000 Leuven (BE)
(72) Inventor: HOSTE, Esther, 9840 De Pinte (BE); MEES, Maarten, 9667 Horebeke (BE); THIELEMANS, Wim, 8500 Kortrijk (BE)
(74) Representative: Brantsandpatents bv
(86) International application number: PCT/EP2019/057215
(87) International publication number: WO 2019/180194

(56) References cited:
- EP-A1- 3 275 447
- WO-A1-2011/111084
- WO-A1-2017/079463
- USAMA FARGHALY ET AL: "Novel Pharmaceutical Gels Containing Glyccerihizic Acid Ammonium Salt for Chronic Wounds", BRITISH JOURNAL OF PHARMACEUTICAL RESEARCH, vol. 4, no. 5, 14 January 2014 (2014-01-14), pages 654 - 668, XP055595949
- K. KOGA ET AL: "Clove oil prevents glycyrrhizin gel formation in aqueous solution", CHEMICAL AND PHARMACEUTICAL BULLETIN, vol. 52, no. 12, December 2004 (2004-12-01), pages 1507 - 1510, XP002787344
- YOSHIOKA ET AL: "Kinetics of the gel-sol transition of the aqueous solutions of @b-glycyrrhizin studied by the temperature jump-spin probe method", JOURNAL OF COLLOID AND INTERFACE SCIENCE, ACADEMIC PRESS,INC, US, vol. 105, no. 1, 1 May 1985 (1985-05-01), pages 65 - 72, XP024207895, ISSN: 0021-9797, [retrieved on 19850501], DOI: 10.1016/0021-9797(85)90347-9

## Description

### Field of the invention

The present invention relates to the field of tissue regeneration, particularly the field of skin lesions such as wounds. The invention provides wound healing compositions. Specifically, the invention provides hydrogel compositions of glycyrrhizic acid. The compositions and methods of the invention are useful especially for assisting the process of wound healing, particularly chronic open lesions that are slow to heal or resistant to healing.

### Introduction to the invention

The skin functions as a protective barrier against environmental threats by acting as the first line of defense. When the skin barrier is compromised, the body is prone to infections and bacteria. Wound healing of the skin consists of five overlapping phases: haemostasis, inflammation, cellular migration, proliferation, and remodelling.^{1,2} Wound healing in mammalian skin results in the formation of a scar, due to the generation of excessive deposits of collagen, a process that is also called fibrosis. The global advanced wound care market size will reach $13.07 billion by 2022 from $10.43 billion in 2017 at a CAGR of 4.6% (during 2017-2022) driven by the increasing prevalence of surgical wounds & ulcers (diabetic foot ulcers, pressure ulcers, and venous leg ulcers), increasing aging population, increasing demand for evidence-based advanced wound care products, rising R&D activities, advancements in the field of specialized wound care research and increasing awareness about wound care treatment according to RnRMarketResearch. A cutaneous wound is defined as damage to the skin by physical or thermal factors or by an infectious agent. Wounds can be separated into two main classes; the first shows no tissue loss (e.g. surgery and cuts) while the second class does show tissue loss (e.g. burn wounds or diabetic ulcers).^{1,3} Both wound types can be further classified based on the duration of repair into either acute (8-12 weeks) or chronic healing (long time and recurring). Chronic wounds, such as venous leg ulcers and pressure ulcers, are common problems in the elderly.¹ As non-healing ulcers often arise post-surgery, they represent a major comorbidity, especially in the aging population. Chronic wounds are often caused by underlying problems such as obesity, diabetes, infections¹, age or ischemia. ⁴⁻⁶ These wounds can evolve into ulcers and even tumours.^{7,8} The current therapy for chronic wound management involves an autograft whereby the patient's own skin is taken from a donor site and grafted on the chronic wound. However, this treatment frequently involves pain and infection at the donor site.¹ The wound care cost for diabetic patients can range from $33,000 to over $50,000 a year.^{8,9} The addition of extra bioactive molecules to a wound can enable faster healing. Indeed, bioactive compounds have been used in the clinic to accelerate and improve wound healing with varying success rate. Therefore, new approaches are needed to improve wound healing, which will have a direct and lasting impact on society both in terms of health as economics. Wound dressings are crucial in wound management because they optimize the healing environment in the wound. Traditional wound dressings such as natural and synthetic bandages, cotton wool, lint and gauzes are still commonly used in the clinic. These dressings were aimed at securing a dry and bacteria-free environment. However, this dry environment is not beneficial for wound healing as a moist environment ensures better healing. Therefore, several wound dressings such as hydrocolloid dressings, alginate dressings, semi-permeable adhesive film hydrogel dressings, foam dressing, biological dressings, tissue engineered skin substitutes, and hydrogels have been developed. There is a current need for fully degradable physical hydrogels as wound dressing in which the gelation agent has anti-inflammatory properties which enables faster wound healing. Hydrogels are defined by IUPAC as *"a gel in which the swelling agent is water".* The gel is formed by a network component, either a polymer, a peptide or a fibril forming a hydrophilic macromolecular network. Both physical or chemical hydrogels can be formed depending on the type of gelation¹⁰. Physical hydrogels or reversible hydrogels are formed by molecular entanglement and/or secondary forces such as hydrogen bonding, Van der Waals and ionic forces. Chemical hydrogels use covalent crosslinking and are more often permanent. Both the physical and chemical hydrogels are used in wound dressing development. Hydrogels show huge potential as wound dressings as they have some striking features in wound healing. Hydrogels contain mainly water, resulting in the rehydration of tissue and cleansing of the wounds, enabling autolytic debridement.¹¹ Malleable hydrogels can absorb exudate, thereby decreasing inflammation and microbial attack. Hydrogels generally consist of at least 90% water, this moist environment delays desiccation of the wound and reduces pain by cooling the surface. Hydrogels also are easily removed because they are non-adherent, thereby reducing pain during removal. However, the current disadvantages of existing hydrogels are that they are semi-transparent and often show poor bacterial resistance. They also often lack poor mechanical stability preventing wider applicability as a secondary cover is needed to protect the wound.⁶ Despite these disadvantages several hydrogels have been commercialized; *Geliperm*^{®}, *Curasol*^{®}*, Tegagel*^{®12} *,Cultinova Gel, Biolex, Tegaderm wound filler^{™} ,* Woulgan and ActiForm cool. Some of them are currently used in the clinic for painful ulcers (*e.g. Actiform cool*). The addition of bioactive molecules to hydrogels can improve wound healing and overcome some of their disadvantages. The regular addition of extra bioactive molecules to a wound can enable faster healing.

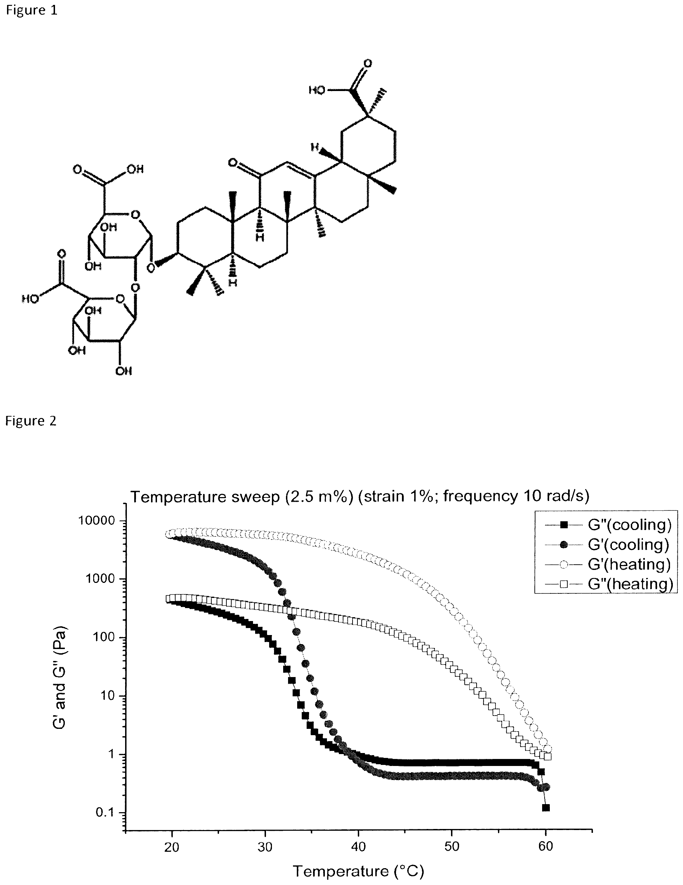

A well-known biological compound is glycyrrhizin, the major compound of liquorice. Glycyrrhizin (herein further abbreviated as Gly) contains a hydrophilic glucuronic acid part and a hydrophobic 18ß-glycyrrhetinic acid terpenoid element (further herein abbreviated as GA) (see Figure 1 for the structure). The terpenoid part is known to exhibit anti-inflammatory properties.¹³ For over 4000 years it was used as a sweetener,¹³ but it is also biologically active as an anticancer agent and it has proven anti-viral^{14,15} and anti-inflammatory properties.¹⁶ It has even been shown that Gly inhibits HIV¹⁷ and reduces asthma-related inflammation¹⁸. In Japan, Gly is used to treat chronic hepatitis B and C infections.¹⁹ In the context of cancer and wound healing, glycyrrhizin inhibits the release of the danger-associated molecular pattern high mobility group box 1 (HMGB1).^{20,21} This inhibitory activity of glycyrrhizin involves the inhibition of the cytokine activities of HMGB1.²² HMGB1 is an important pro-inflammatory molecule and is known to be involved in tissue remodelling and angiogenesis.²³It was found that the Gly interferes with the amino acid residues Lys^{90,} Arg⁹¹, Ser¹⁰¹, Tyr¹⁴⁹, C²³⁰, C²³¹ and thereby inhibits HMGB1.²⁴Gly was used as a therapeutic for treating HMGB1-involved diseases such as: microbial keratitis²⁵.

EP3275447A1 discloses gels comprising Gly, water and an anesthetic agent to reduce pain in wound areas. The anesthetic agent is required in the composition to form a gel. The application WO2011111084A1 teaches pharmaceutical compositions containing the thermosensitive polymer EG56 (which is bis-methoxy PEG-13 PEG-438/PPG-110 SMDI copolymer) and glycyrrhizin or glycyrrhetinic acid which are used for the treatment of inflammation in the eye and nasal deposition. In our experimental conditions we witnessed that by mixing the polymer (EG56) and Gly, a hydrogel is formed which is not thermoresponsive. The loss in thermoresponsivity is caused by the 18β-glycyrrhetinic acid part of the Gly, which forms thermoreversible viscoelastic gels in different kind of solvents.²⁶ The gelation of Gly (in an aqueous composition) occurs due to intermolecular hydrogen bonding and the van der Waals interaction of the triterpenoid backbone. The third interaction is the dipole-dipole interaction of the deprotonated carboxyl group, which is only found in the salt form. Our examples show that a hydrogel of Gly and EG56 causes more inflammation and reduces wound healing. Gly hydrogels are used in the synthesis of gold nanoparticles were they act as a template^{27,28} and glycyrrhetinic acid hydrogels have a potential for the removal of organic dyes²⁹ and even emulsify vegetable oils.³⁰ Yoshioka H. (1984) Journal of Colloid and Interface Science, 105(1), 65 describes the properties of Gly solutions in order to use it as a nontoxic solubilizer or emulsifier and teaches the gelation properties of a 9.2% Gly aqueous solution.

USAMA FARGHALY ET AL: "Novel Pharmaceutical Gels Containing Glyccerihizic Acid Ammonium Salt for Chronic Wounds",BRITISH JOURNAL OF PHARMACEUTICAL RESEARCH, vol. 4, no. 5, 14 January 2014 (2014-01-14), pages 654-668, discloses gels comprising 3% glycyrrhizic acid for use in treating wounds, including chronic wounds.

### Summary of the invention

The present invention provides wound healing compositions of a degradable biocompatible anti-inflammatory wound dressing for topical application to an external wound of a mammal as defined in claim 1. In particular, the invention provides topical plasters comprising a hydrogel essentially consisting of a molecule with formula (I) and water as defined in claim 4. In the present disclosure we show that hydrogels are formed by gelation of 2,5%-25% of glycyrrhizic acid (Gly) and variants thereof as defined in claim 1 in water, meaning that the only components needed to make the hydrogel are water and Gly or a variant thereof. Importantly, the exemplified hydrogels have the capacity to maintain a moist environment, take up exudate and are fully degradable. In addition, Gly is non-toxic and FDA approved as a food additive. With its double function as a gel-forming agent and anti-inflammatory agent, at 2,5%-25% by weight of glycyrrhizin in water it forms an ideal hydrogel to develop a new kind of wound dressing that greatly improves wound healing. Advantageously compared to other hydrogels there is no need for an additional gelator, as Gly acts as the gelator, thereby reducing toxicity and avoiding an immune response. Because Gly is water soluble, no organic solvents are required during synthesis of the hydrogel, again reducing toxicity.

### Figures

Figure 1: chemical structure of glycyrrhizin
Figure 2: Temperature sweep (20-60°C, rate 0.02°C/s) of the 2.5% Gly hydrogel. Strain (1%) and frequency (10 rad/s) indicating the gelation point at 40°C (the filled red (circles) and filled black (squares) lines cross, cooling ramp)
Figure 3: Temperature sweep (20-60°C, 0.02 rad/s) of the 5% Gly hydrogel. Strain (1%) and frequency (10 rad/s) indicating the gelation point at 46°C (the filled red (circles) and black (squares) lines cross, cooling ramp)
Figure 4: Strain sweep (10 rad/s) (lower part) and frequency sweep (0.5%) (upper part) of the 2.5 % Gly hydrogel at 37°C
Figure 5: Strain sweep (10 rad/s) (upper part) and frequency sweep (0.025%) (lower part) of the 5 % Gly hydrogel at 37°C
Figure 6: Stress sweep for the two types of hydrogel (2.5 % Gly (left part of the picture) and 5% Gly (right part of the picture)
Figure 7: Temperature sweep (Gly 2m% (strain 1% and freq. 10 rad/s). The heating curve is indicated by the hollow symbols while the cooling curve is indicated by filled symbols.
Figure 8: Temperature sweep (Gly 2.5m% (first panel), 5.6 m% (second panel), 5m % (third panel) (strain 1% and freq. 10 rad/s). The heating curve is indicated by the hollow symbols while the cooling curve is indicated by filled symbols. (G'>G" at 43°C)
Figure 9: Temperature sweep (Gly 9.1 m% (first panel), Gly 13 m% (second panel), Gly 20 m% (third panel)) (strain 0.006% and freq. 10 rad/s) The heating curves are indicated by the hollow symbols while the cooling curve are indicated by filled symbols.
Figure 10: (a) Wound healing rates in mice untreated or treated with 5% Gly hydrogel (Glyc) or PEG (n= 9 mice per group), (b) percentage of mice with fully-closed wounds at day 12 post-wounding (Glyc: 5% Gly hydrogel, untr: untreated and PEG: EG56/5% Gly composition), (c) Representative pictures of full-thickness skin wounds at the day of wounding (day 0), day 2 and day 4 post-wounding.
Figure 11: Kinetics of wound closure in normoglycemic murine conditions. One 8mm full-thickness skin wound was inflicted per mouse on the back skin. Wounds were treated with 4 different hydrogels. It is apparent that a hydrogel consisting of 10% glycyrrhizin is the most performant hydrogel. The 5% glycyrrhizin hydrogel also outperforms Tegaderm and Woulgan hydrogels. Histograms represent means ± SEM, data were analyzed by Two-way ANOVA with multiple comparisons.
Figure 12: percentage of mice with closed wounds over a period of 8 to 14 days post-wounding. * P<0.5 Log Rank (Mantel-Cox test).
Figure 13: pictures of wounds induced in normoglycemic mice and treated with the 4 different hydrogels.
Figure 14: Kinetics of wound closure in diabetic mice treated with 4 different hydrogel compositions. Histograms represent means ± SEM, data were analyzed by Two-way ANOVA with multiple comparisons.
Figure 15: wound closure in diabetic mice treated with different hydrogel compositions. Data was analyzed by Log Rank (Mantel-Cox) testing.
Figure 16: pictures of wounds inflicted on diabetic mice and treated with the 4 different hydrogels
Figure 17: wound closure in pigs treated with different hydrogels over a period of 14 days . Histograms represent means ± SEM, data were analyzed by Two-way ANOVA with multiple comparisons.
Figure 18: wound sizes in pigs after a period of 14 days after treatment with different hydrogels. Wounds were measured at the indicated time-points, histograms represent mean ± SEM (Two-way ANOVA; * 0.01<P<0.05; ** 0.001<P<0,01; *** 0.0001<P<0.001).
Figure 19: Percentage of closed cutaneous wounds at day 14 post-wounding (d14 post wounding (pw); n=8 wounds per treatment condition).
Figure 20: Representative pictures of cutaneous porcine wounds at the indicated time-point and treatment effects with different hydrogels
Figure 21: Rate of wound healing of murine skin treated with different hydrogels (n=10 per condition). Wounds were treated every other day with 500 µl Tegaderm wound filler (Tegaderm^{™}), 5% Glycyrrhetinic acid (5% GA) or 15% Glycyrrhetinic acid (15% GA). Wounds were measured at the indicated time-points. Histograms represent mean ± SEM (Two-way ANOVA; * 0.01<P<0.05; **** P<0.0001).

### Detailed description of the invention

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn to scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. Unless specifically defined herein, all terms used herein have the same meaning as they would to one skilled in the art of the present invention. The definitions provided herein should not be construed to have a scope less than understood by a person of ordinary skill in the art.

The present invention provides hydrogels which are optimal wound dressings creating a moist, clean and warm environment meeting the following properties:
➢ ensuring a moist wound environment as this prevents desiccation and corresponding necrosis, increasing epidermal migration and promoting angiogenesis.
➢ capable of removing excess exudate from the wound. This is important in chronic wounds as the exudate contains tissue-degrading enzymes that block proliferation and macerate the healthy surrounding tissue.¹¹
➢ providing prevention against microorganisms, infection and contamination.
➢ stimulating the production of growth factors.
➢ providing mechanical protection while being elastic enough to be easily applied and removed with minimal pain.
➢ being hypoallergenic and non-toxic.
➢ being biocompatible/biodegradable.
➢ being easily sterilisable.

Accordingly, the present invention provides in a first embodiment a topical plaster comprising a hydrogel essentially consisting of 3,5%-25% of glycyrrhizin and water for use as a medicament.

The wording "5% of a molecule" (such as Gly) as described herein is equivalent to "m5% of a molecule" (such as Gly) and refers to 5g of a molecule (such as Gly) dissolved in 100 gram of water (H₂O).

In yet another embodiment the invention provides a hydrogel consisting of 5%-25% of glycyrrhizic acid and water for use as a medicament according to claim 1.

In yet another embodiment the invention provides a hydrogel consisting of 5%-20% of glycyrrhizic acid and water for use as a medicament according to claim 1.

In yet another embodiment the invention provides a hydrogel consisting of 5%-15% of glycyrrhizic acid and water for use as a medicament according to claim 1.

In yet another embodiment the invention provides a hydrogel consisting of 5%-10% of glycyrrhizic acid and water for use as a medicament according to claim 1.

In yet another embodiment the invention provides a hydrogel essentially consisting of 5%-20% of glycyrrhizic acid and water for use as a medicament according to claim 1.

In yet another embodiment the invention provides a hydrogel essentially consisting of 5%-15% of glycyrrhizic acid and water for use as a medicament according to claim 1.

In yet another embodiment the invention provides a hydrogel essentially consisting of 5%-10% of glycyrrhizic acid and water for use as a medicament according to claim 1.

In yet another embodiment the present invention provides a hydrogel consisting of 5%-20% of glycyrrhizic acid and water, which has a temperature lower than 40°C for use as a medicament according to claim 1.

In yet another embodiment the present invention provides a hydrogel consisting of 5%-15% of glycyrrhizic acid and water, which has a temperature lower than 40°C for use as a medicament according to claim 1.

In yet another embodiment the present invention provides a hydrogel essentially consisting of 5%-15% of glycyrrhizic acid and water, which has a temperature lower than 40°C for use as a medicament according to claim 1.

It is essential that the degelation point of the hydrogel is higher than the body temperature of the mammal to be treated for a wound otherwise the hydrogel will become liquid when applied onto the wound. For example the gelation point for the 2.5% Gly hydrogel was determined to be 40°C while the gelation point for the 5% Gly hydrogel was determined to be at 45°C (see Example 1 in this application).

In polymer chemistry, the gelation point (or gel point) is an abrupt change in the viscosity of a solution containing polymerizable components. At the gelation point, a solution undergoes gelation as reflected in a loss in fluidity. Conversely, the degelation point of a solution is the opposite as the gelation point and refers to the point (or the temperature) where a solution undergoes a gain in fluidity.

Also disclosed is a hydrogel consisting of 2.5%-3%, 2.5%-3.5%, 2.5%-4%, 2.5%-4.5%, 2.5%-5%, 2.5%-5.5%, 2.5%-6%, 2.5%-6.5%, 2.5%-7%, 2.5%-7.5%, 2.5%-8%, 2.5%-8.5%, 2.5%-9%, 2.5%-9.5%, 2.5%-10%, 2.5%--10,5%, 2.5%-11%, 2.5%-11,5%, 2.5%-12%, 2.5%-12.5%, 2.5%-13%, 2.5%--13.5%, 2.5%-14%, 2.5%-14.5%, 2.5%-15%, 2.5%-16%, 2.5%-17%, 2.5%-18%, 2.5%-19%, 2.5%-20%, 2.5%-21%, 2.5%-22%, 2.5%-23%, 2.5%-24%, 2.5%-25%, 3%-3.5%, 3%-4%, 3%-4.5%, 3%-5%, 3%-5.5%, 3%-6%, 3%-6.5%, 3%-7%, 3%-7.5%, 3%-8%, 3%-8.5%, 3%-9%, 3%-9.5%, 3%-10%, 3%-10.5%, 3%-11%, 3%-11.5%, 3%-12%, 3%-12.5%, 3%-13%, 3%-13.5%, 3%-14%, 3%-14.5%, 3%-15%, 3%-16%, 3%-17%, 3%-18%, 3%-19%, 3%-20%, 3%-21%, 3%-22%, 3%-23%, 3%-24%, 3%-25%, 3.5%-4%, 3.5%-4.5%, 3.5%-5%, 3.5%-5.5%, 3.5%-6%, 3.5%-6.5%, 3.5%-7%, 3.5%-7.5%, 3.5%-8%, 3.5%-8.5%, 3.5%-9%, 3.5%-9.5%, 3.5%-10%, 3.5%-10.5%, 3.5%-11%, 3.5%-11.5%, 3.5%-12%, 3.5%-12.5%, 3.5%-13%, 3.5%-13.5%, 3.5%-14%, 3.5%-14.5%, 3.5%-15%, 3.5%-16%, 3.5%-17%, 3.5%-18%, 3.5%-19%, 3.5%-20%, 3.5%-21%, 3.5%-22%, 3.5%-23%, 3.5%-24%, 3.5%-25%, 4%-4.5%, 4%-5%, 4%-5.5%, 4%-6%, 4%-6.5%, 4%-7%, 4%-7.5%, 4%-8%, 4%-8.5%, 4%-9%, 4%-9.5%, 4%-10%, 4%-10.5%, 4%-11%, 4%-11.5%, 4%-12%, 4%-12.5%, 4%-13%, 4%-13.5%, 4%-14%, 4%-14.5%, 4%-15%, 4%-16%, 4%-17%, 4%-18%, 4%-19%, 4%-20%, 4%-21%, 4%-22%, 4%-23%, 4%-24%, 4%-25%, 4.5%-5%, 4.5%-5.5%, 4.5%-6%, 4.5%-6.5%, 4.5%-7%, 4.5%-7.5%, 4.5%-8%, 4.5%-8.5%, 4.5%-9%, 4.5%-9.5%, 4.5%-10%, 4.5%-10.5%, 4.5%-11%, 4.5%-11.5%, 4.5%-12%, 4.5%-12.5%, 4.5%-13%, 4.5%-13.5%, 4.5%-14%, 4.5%-14.5%, 4.5%-15%, 4.5%-16%, 4.5%-17%, 4.5%-18%, 4.5%-19%, 4.5%-20%, 4.5%-21%, 4.5%-22%, 4.5%-23%, 4.5%-24%, 4.5%-25%, 5%-5.5%, 5%-6%, 5%-6.5%, 5%-7%, 5%-7.5%, 5%-8%, 5%-8.5%, 5%-9%, 5%-9.5%, 5%-10%, 5%-10.5%, 5%-11%, 5%-11.5%, 5%-12%, 5%-12.5%, 5%-13%, 5%-13.5%, 5%-14%, 5%-14.5%, 5%-15%, 5%-16%, 5%-17%, 5%-18%, 5%-19%, 5%-20%, 5%-21%, 5%-22%, 5%-23%, 5%-24%, 5%-25%, 6%-7%, 6%-8%, 6%-9%, 6%-10%, 6%-11%, 6%-12%, 6%-13%, 6%-14%, 6%-15%, 6%-16%, 6%-17%, 6%-18%, 6%-19%, 6%-20%, 6%-21%, 6%-22%, 6%-23%, 6%-24%, 6%-25%, 7%-8%, 7%-9%, 7%-10%, 7%-11%, 7%-12%, 7%-13%, 7%-14%, 7%-15%, 7%-16%, 7%-17%, 7%-18%, 7%-19%, 7%-20%, 7%-21%, 7%-22%, 7%-23%, 7%-24%, 7%-25%, 8%-9%, 8%-10%, 8%-11%, 8%-12%, 8%-13%, 8%-14%, 8%-15%, 8%-16%, 8%-17%, 8%-18%, 8%-19%, 8%-20%, 8%-21%, 8%-22%, 8%-23%, 8%-24%, 8%-25%, 9%-10%, 9%-11%, 9%-12%, 9%-13%, 9%-14%, 9%-15%, 9%-16%, 9%-17%, 9%-18%, 9%-19%, 9%-20%, 9%-21%, 9%-22%, 9%-23%, 9%-24%, 9%-25%, 10%-12%, 10%-14%, 10%-16%, 10%-18%, 10%-20%, 10%-22%, 10%-25%, 12%-15%, 12%-18%, 12%-21%, 12%-25%, 14%-18%, 14%-21%, 14%-23%, 14%-25%, 16%-19%, 16%-21%, 16%-23%, 16%-25%, or 25% of glycyrrhizic acid and water for use as a medicament. Embodiments with an endpoint outside of the claimed 3,5%-25% range are not part of the invention.

In a specific embodiments, not part of the invention the hydrogels of the invention include an anti-microbial compound for use as a medicament.

Anti-microbial compounds are for example water soluble antibiotics (ciproflaxacin, cefazolin, Neosporin^{®}), anti-cancer drugs such as 5-fluorouracil, antimicrobials (iodine silver), model compounds (fluorescein containing dextran), anti-fungal compounds and singlet oxygen generating molecules for chemical ablation of bacteria (Rose Bengal, Methylene Blue). For example, the addition of antibiotics to the hydrogels is potentially useful in chronic wounds.

Chronic wounds often do not benefit from treatment with systemic antibiotic as the supply of antibiotics to the wound site is low due to lack of blood supply.

In yet other specific embodiments, not part of the invention the hydrogels of the invention include a biocompatible polymer, with the exception of EG56, for use as a medicament.

In specific concentrations of Gly in the hydrogels of the invention it might be useful to add biocompatible polymers to the Gly hydrogel ensuring a stronger network. Indeed, one of the possible drawbacks of the use of specific hydrogels is the low mechanical strength and often a secondary bandage is needed. Thus, for certain wounds which are in contact areas it can be useful to increase the mechanical strength. To overcome the low mechanical strength of the hydrogels, a polymer is introduced to reinforce the hydrogel and improve both mechanical and rheological properties. This will be done by making an interpenetrating polymer network, this is the interlacing of two polymer networks without covalent linking. Therefore, biocompatible polymers, excluding EG56, are added to the Gly hydrogel to ensure a stronger network. Preferred biocompatible polymers to add to the hydrogels of the invention are chitosan, cellulose, poly(2-alkyl-oxazoline)s, polyvinylpyrrolidione, polyacrylicacid, polyacrylamide, polyphosphates, polyphosphazene, xanthan, pectines, dextran and poly(vinyl alcohol)

In yet other specific embodiments, not part of the invention the hydrogels of the invention include an anti-inflammatory agent for use as a medicament.

In a specific embodiment the anti-inflammatory agent is a glucocorticoid. Glucocorticoids are known to have a biological activity on wound healing responses, scar formation and prevention of tumour formation in the skin.

In yet other specific embodiments, not part of the invention the hydrogels of the invention include an anti-microbial compound and a biocompatible polymer for use as a medicament.

In yet another specific embodiment, not part of the invention the hydrogels of the invention include an anti-microbial compound, a biocompatible polymer and an anti-inflammatory compound for use as a medicament.

The skilled person active in the field of material characterization knows that by adding anti-microbial compounds and/or a biocompatible polymer and/or an anti-inflammatory compound to the Gly hydrogels that the hydrogel formation can be disturbed and that an optimal concentration of Gly (id est ranges as described herein before between 2,5%-25% of Gly) varies depending on the amount and type of additional molecules added to the hydrogels of the invention. Therefore, these hydrogels containing additional molecules are screened for the same properties as the Gly hydrogels consisting of water and a concentration between 2,5%-25% Gly, being mechanical properties, rheological behaviour, the water release and exudate uptake. The possible distortion of gel formation is monitored via polarised microscopy and by SAXS (Small Angle X-ray Scattering) and WAXS (Wide Angle X-ray Scattering). Evidently, a particular compound will not be added to the Gly hydrogel when there is insufficient hydrogel formation, the gelation is below body temperature or if the active compound concentration cannot reach the pharmaceutical window to be of clinical relevance. Here, it is important to investigate what the interaction is between the polymers and the Gly fibril and how they can reinforce the Gly hydrogels. Therefore, we will use polarised microscopy and SAXS (small-angle X-ray scattering) and WAXS (wide angel scattering). Furthermore, the hydrogel will be tested on mechanical properties, rheological behaviour, water release and exudate uptake to find the strongest hydrogel for wound healing applications. WAXS and SAXS are non-destructive X-ray diffraction techniques which can be used to determine the crystalline structure of polymers³¹ WAXS is the same technique as SAXS, only the distance from sample to the detector is shorter and thus diffraction maxima at larger angles are observed. Depending on the measurement instrument used it is possible to perform WAXS and SAXS in a single run (small- and wide-angle scattering, SWAXS).

In yet other specific embodiments the hydrogels of the invention for use as a medicament have a pH range between 5 and 8.

Also disclosed but not part of the invention is an aqueous hydrogel comprising 2.5%-3%, 2.5%-3.5%, 2.5%-4%, 2.5%-4.5%, 2.5%-5%, 2.5%-5.5%, 2.5%-6%, 2.5%-6.5%, 2.5%-7%, 2.5%-7.5%, 2.5%-8%, 2.5%-8.5%, 2.5%-9%, 2.5%-9.5%, 2.5%-10%, 2.5%--10,5%, 2.5%-11%, 2.5%-11,5%, 2.5%-12%, 2.5%-12.5%, 2.5%-13%, 2.5%--13.5%, 2.5%-14%, 2.5%-14.5%, 2.5%-15%, 2.5%-16%, 2.5%-17%, 2.5%-18%, 2.5%-19%, 2.5%-20%, 2.5%-21%, 2.5%-22%, 2.5%-23%, 2.5%-24%, 2.5%-25%, 3%-3.5%, 3%-4%, 3%-4.5%, 3%-5%, 3%-5.5%, 3%-6%, 3%-6.5%, 3%-7%, 3%-7.5%, 3%-8%, 3%-8.5%, 3%-9%, 3%-9.5%, 3%-10%, 3%-10.5%, 3%-11%, 3%-11.5%, 3%-12%, 3%-12.5%, 3%-13%, 3%-13.5%, 3%-14%, 3%-14.5%, 3%-15%, 3%-16%, 3%-17%, 3%-18%, 3%-19%, 3%-20%, 3%-21%, 3%-22%, 3%-23%, 3%-24%, 3%-25%, 3.5%-4%, 3.5%-4.5%, 3.5%-5%, 3.5%-5.5%, 3.5%-6%, 3.5%-6.5%, 3.5%-7%, 3.5%-7.5%, 3.5%-8%, 3.5%-8.5%, 3.5%-9%, 3.5%-9.5%, 3.5%-10%, 3.5%-10.5%, 3.5%-11%, 3.5%-11.5%, 3.5%-12%, 3.5%-12.5%, 3.5%-13%, 3.5%-13.5%, 3.5%-14%, 3.5%-14.5%, 3.5%-15%, 3.5%-16%, 3.5%-17%, 3.5%-18%, 3.5%-19%, 3.5%-20%, 3.5%-21%, 3.5%-22%, 3.5%-23%, 3.5%-24%, 3.5%-25%, 4%-4.5%, 4%-5%, 4%-5.5%, 4%-6%, 4%-6.5%, 4%-7%, 4%-7.5%, 4%-8%, 4%-8.5%, 4%-9%, 4%-9.5%, 4%-10%, 4%-10.5%, 4%-11%, 4%-11.5%, 4%-12%, 4%-12.5%, 4%-13%, 4%-13.5%, 4%-14%, 4%-14.5%, 4%-15%, 4%-16%, 4%-17%, 4%-18%, 4%-19%, 4%-20%, 4%-21%, 4%-22%, 4%-23%, 4%-24%, 4%-25%, 4.5%-5%, 4.5%-5.5%, 4.5%-6%, 4.5%-6.5%, 4.5%-7%, 4.5%-7.5%, 4.5%-8%, 4.5%-8.5%, 4.5%-9%, 4.5%-9.5%, 4.5%-10%, 4.5%-10.5%, 4.5%-11%, 4.5%-11.5%, 4.5%-12%, 4.5%-12.5%, 4.5%-13%, 4.5%-13.5%, 4.5%-14%, 4.5%-14.5%, 4.5%-15%, 4.5%-16%, 4.5%-17%, 4.5%-18%, 4.5%-19%, 4.5%-20%, 4.5%-21%, 4.5%-22%, 4.5%-23%, 4.5%-24%, 4.5%-25%, 5%-5.5%, 5%-6%, 5%-6.5%, 5%-7%, 5%-7.5%, 5%-8%, 5%-8.5%, 5%-9%, 5%-9.5%, 5%-10%, 5%-10.5%, 5%-11%, 5%-11.5%, 5%-12%, 5%-12.5%, 5%-13%, 5%-13.5%, 5%-14%, 5%-14.5%, 5%-15%, 5%-16%, 5%-17%, 5%-18%, 5%-19%, 5%-20%, 5%-21%, 5%-22%, 5%-23%, 5%-24%, 5%-25%, 6%-7%, 6%-8%, 6%-9%, 6%-10%, 6%-11%, 6%-12%, 6%-13%, 6%-14%, 6%-15%, 6%-16%, 6%-17%, 6%-18%, 6%-19%, 6%-20%, 6%-21%, 6%-22%, 6%-23%, 6%-24%, 6%-25%, 7%-8%, 7%-9%, 7%-10%, 7%-11%, 7%-12%, 7%-13%, 7%-14%, 7%-15%, 7%-16%, 7%-17%, 7%-18%, 7%-19%, 7%-20%, 7%-21%, 7%-22%, 7%-23%, 7%-24%, 7%-25%, 8%-9%, 8%-10%, 8%-11%, 8%-12%, 8%-13%, 8%-14%, 8%-15%, 8%-16%, 8%-17%, 8%-18%, 8%-19%, 8%-20%, 8%-21%, 8%-22%, 8%-23%, 8%-24%, 8%-25%, 9%-10%, 9%-11%, 9%-12%, 9%-13%, 9%-14%, 9%-15%, 9%-16%, 9%-17%, 9%-18%, 9%-19%, 9%-20%, 9%-21%, 9%-22%, 9%-23%, 9%-24%, 9%-25%, 10%-12%, 10%-14%, 10%-16%, 10%-18%, 10%-20%, 10%-22%, 10%-25%, 12%-15%, 12%-18%, 12%-21%, 12%-25%, 14%-18%, 14%-21%, 14%-23%, 14%-25%, 16%-19%, 16%-21%, 16%-23%, 16%-25%, or 25% of glycyrrhizic acid and water for use to treat wound healing. These embodiments are not encompassed by the wording of the claims.

Also disclosed is an aqueous hydrogel consisting of 2.5%-3%, 2.5%-3.5%, 2.5%-4%, 2.5%-4.5%, 2.5%-5%, 2.5%-5.5%, 2.5%-6%, 2.5%-6.5%, 2.5%-7%, 2.5%-7.5%, 2.5%-8%, 2.5%-8.5%, 2.5%-9%, 2.5%-9.5%, 2.5%-10%, 2.5%--10,5%, 2.5%-11%, 2.5%-11,5%, 2.5%-12%, 2.5%-12.5%, 2.5%-13%, 2.5%--13.5%, 2.5%-14%, 2.5%-14.5%, 2.5%-15%, 2.5%-16%, 2.5%-17%, 2.5%-18%, 2.5%-19%, 2.5%-20%, 2.5%-21%, 2.5%-22%, 2.5%-23%, 2.5%-24%, 2.5%-25%, 3%-3.5%, 3%-4%, 3%-4.5%, 3%-5%, 3%-5.5%, 3%-6%, 3%-6.5%, 3%-7%, 3%-7.5%, 3%-8%, 3%-8.5%, 3%-9%, 3%-9.5%, 3%-10%, 3%-10.5%, 3%-11%, 3%-11.5%, 3%-12%, 3%-12.5%, 3%-13%, 3%-13.5%, 3%-14%, 3%-14.5%, 3%-15%, 3%-16%, 3%-17%, 3%-18%, 3%-19%, 3%-20%, 3%-21%, 3%-22%, 3%-23%, 3%-24%, 3%-25%, 3.5%-4%, 3.5%-4.5%, 3.5%-5%, 3.5%-5.5%, 3.5%-6%, 3.5%-6.5%, 3.5%-7%, 3.5%-7.5%, 3.5%-8%, 3.5%-8.5%, 3.5%-9%, 3.5%-9.5%, 3.5%-10%, 3.5%-10.5%, 3.5%-11%, 3.5%-11.5%, 3.5%-12%, 3.5%-12.5%, 3.5%-13%, 3.5%-13.5%, 3.5%-14%, 3.5%-14.5%, 3.5%-15%, 3.5%-16%, 3.5%-17%, 3.5%-18%, 3.5%-19%, 3.5%-20%, 3.5%-21%, 3.5%-22%, 3.5%-23%, 3.5%-24%, 3.5%-25%, 4%-4.5%, 4%-5%, 4%-5.5%, 4%-6%, 4%-6.5%, 4%-7%, 4%-7.5%, 4%-8%, 4%-8.5%, 4%-9%, 4%-9.5%, 4%-10%, 4%-10.5%, 4%-11%, 4%-11.5%, 4%-12%, 4%-12.5%, 4%-13%, 4%-13.5%, 4%-14%, 4%-14.5%, 4%-15%, 4%-16%, 4%-17%, 4%-18%, 4%-19%, 4%-20%, 4%-21%, 4%-22%, 4%-23%, 4%-24%, 4%-25%, 4.5%-5%, 4.5%-5.5%, 4.5%-6%, 4.5%-6.5%, 4.5%-7%, 4.5%-7.5%, 4.5%-8%, 4.5%-8.5%, 4.5%-9%, 4.5%-9.5%, 4.5%-10%, 4.5%-10.5%, 4.5%-11%, 4.5%-11.5%, 4.5%-12%, 4.5%-12.5%, 4.5%-13%, 4.5%-13.5%, 4.5%-14%, 4.5%-14.5%, 4.5%-15%, 4.5%-16%, 4.5%-17%, 4.5%-18%, 4.5%-19%, 4.5%-20%, 4.5%-21%, 4.5%-22%, 4.5%-23%, 4.5%-24%, 4.5%-25%, 5%-5.5%, 5%-6%, 5%-6.5%, 5%-7%, 5%-7.5%, 5%-8%, 5%-8.5%, 5%-9%, 5%-9.5%, 5%-10%, 5%-10.5%, 5%-11%, 5%-11.5%, 5%-12%, 5%-12.5%, 5%-13%, 5%-13.5%, 5%-14%, 5%-14.5%, 5%-15%, 5%-16%, 5%-17%, 5%-18%, 5%-19%, 5%-20%, 5%-21%, 5%-22%, 5%-23%, 5%-24%, 5%-25%, 6%-7%, 6%-8%, 6%-9%, 6%-10%, 6%-11%, 6%-12%, 6%-13%, 6%-14%, 6%-15%, 6%-16%, 6%-17%, 6%-18%, 6%-19%, 6%-20%, 6%-21%, 6%-22%, 6%-23%, 6%-24%, 6%-25%, 7%-8%, 7%-9%, 7%-10%, 7%-11%, 7%-12%, 7%-13%, 7%-14%, 7%-15%, 7%-16%, 7%-17%, 7%-18%, 7%-19%, 7%-20%, 7%-21%, 7%-22%, 7%-23%, 7%-24%, 7%-25%, 8%-9%, 8%-10%, 8%-11%, 8%-12%, 8%-13%, 8%-14%, 8%-15%, 8%-16%, 8%-17%, 8%-18%, 8%-19%, 8%-20%, 8%-21%, 8%-22%, 8%-23%, 8%-24%, 8%-25%, 9%-10%, 9%-11%, 9%-12%, 9%-13%, 9%-14%, 9%-15%, 9%-16%, 9%-17%, 9%-18%, 9%-19%, 9%-20%, 9%-21%, 9%-22%, 9%-23%, 9%-24%, 9%-25%, 10%-12%, 10%-14%, 10%-16%, 10%-18%, 10%-20%, 10%-22%, 10%-25%, 12%-15%, 12%-18%, 12%-21%, 12%-25%, 14%-18%, 14%-21%, 14%-23%, 14%-25%, 16%-19%, 16%-21%, 16%-23%, 16%-25%, or 25% of glycyrrhizic acid and water for use to treat wound healing. Embodiments with an endpoint outside of the claimed 3,5%-25% are not encompassed by the wording of the claims.

Also disclosed but not part of the invention is an aqueous hydrogel consisting of 2.5%-3%, 2.5%-3.5%, 2.5%-4%, 2.5%-4.5%, 2.5%-5%, 2.5%-5.5%, 2.5%-6%, 2.5%-6.5%, 2.5%-7%, 2.5%-7.5%, 2.5%-8%, 2.5%-8.5%, 2.5%-9%, 2.5%-9.5%, 2.5%-10%, 2.5%--10,5%, 2.5%-11%, 2.5%-11,5%, 2.5%-12%, 2.5%-12.5%, 2.5%-13%, 2.5%--13.5%, 2.5%-14%, 2.5%-14.5%, 2.5%-15%, 2.5%-16%, 2.5%-17%, 2.5%-18%, 2.5%-19%, 2.5%-20%, 2.5%-21%, 2.5%-22%, 2.5%-23%, 2.5%-24%, 2.5%-25%, 3%-3.5%, 3%-4%, 3%-4.5%, 3%-5%, 3%-5.5%, 3%-6%, 3%-6.5%, 3%-7%, 3%-7.5%, 3%-8%, 3%-8.5%, 3%-9%, 3%-9.5%, 3%-10%, 3%-10.5%, 3%-11%, 3%-11.5%, 3%-12%, 3%-12.5%, 3%-13%, 3%-13.5%, 3%-14%, 3%-14.5%, 3%-15%, 3%-16%, 3%-17%, 3%-18%, 3%-19%, 3%-20%, 3%-21%, 3%-22%, 3%-23%, 3%-24%, 3%-25%, 3.5%-4%, 3.5%-4.5%, 3.5%-5%, 3.5%-5.5%, 3.5%-6%, 3.5%-6.5%, 3.5%-7%, 3.5%-7.5%, 3.5%-8%, 3.5%-8.5%, 3.5%-9%, 3.5%-9.5%, 3.5%-10%, 3.5%-10.5%, 3.5%-11%, 3.5%-11.5%, 3.5%-12%, 3.5%-12.5%, 3.5%-13%, 3.5%-13.5%, 3.5%-14%, 3.5%-14.5%, 3.5%-15%, 3.5%-16%, 3.5%-17%, 3.5%-18%, 3.5%-19%, 3.5%-20%, 3.5%-21%, 3.5%-22%, 3.5%-23%, 3.5%-24%, 3.5%-25%, 4%-4.5%, 4%-5%, 4%-5.5%, 4%-6%, 4%-6.5%, 4%-7%, 4%-7.5%, 4%-8%, 4%-8.5%, 4%-9%, 4%-9.5%, 4%-10%, 4%-10.5%, 4%-11%, 4%-11.5%, 4%-12%, 4%-12.5%, 4%-13%, 4%-13.5%, 4%-14%, 4%-14.5%, 4%-15%, 4%-16%, 4%-17%, 4%-18%, 4%-19%, 4%-20%, 4%-21%, 4%-22%, 4%-23%, 4%-24%, 4%-25%, 4.5%-5%, 4.5%-5.5%, 4.5%-6%, 4.5%-6.5%, 4.5%-7%, 4.5%-7.5%, 4.5%-8%, 4.5%-8.5%, 4.5%-9%, 4.5%-9.5%, 4.5%-10%, 4.5%-10.5%, 4.5%-11%, 4.5%-11.5%, 4.5%-12%, 4.5%-12.5%, 4.5%-13%, 4.5%-13.5%, 4.5%-14%, 4.5%-14.5%, 4.5%-15%, 4.5%-16%, 4.5%-17%, 4.5%-18%, 4.5%-19%, 4.5%-20%, 4.5%-21%, 4.5%-22%, 4.5%-23%, 4.5%-24%, 4.5%-25%, 5%-5.5%, 5%-6%, 5%-6.5%, 5%-7%, 5%-7.5%, 5%-8%, 5%-8.5%, 5%-9%, 5%-9.5%, 5%-10%, 5%-10.5%, 5%-11%, 5%-11.5%, 5%-12%, 5%-12.5%, 5%-13%, 5%-13.5%, 5%-14%, 5%-14.5%, 5%-15%, 5%-16%, 5%-17%, 5%-18%, 5%-19%, 5%-20%, 5%-21%, 5%-22%, 5%-23%, 5%-24%, 5%-25%, 6%-7%, 6%-8%, 6%-9%, 6%-10%, 6%-11%, 6%-12%, 6%-13%, 6%-14%, 6%-15%, 6%-16%, 6%-17%, 6%-18%, 6%-19%, 6%-20%, 6%-21%, 6%-22%, 6%-23%, 6%-24%, 6%-25%, 7%-8%, 7%-9%, 7%-10%, 7%-11%, 7%-12%, 7%-13%, 7%-14%, 7%-15%, 7%-16%, 7%-17%, 7%-18%, 7%-19%, 7%-20%, 7%-21%, 7%-22%, 7%-23%, 7%-24%, 7%-25%, 8%-9%, 8%-10%, 8%-11%, 8%-12%, 8%-13%, 8%-14%, 8%-15%, 8%-16%, 8%-17%, 8%-18%, 8%-19%, 8%-20%, 8%-21%, 8%-22%, 8%-23%, 8%-24%, 8%-25%, 9%-10%, 9%-11%, 9%-12%, 9%-13%, 9%-14%, 9%-15%, 9%-16%, 9%-17%, 9%-18%, 9%-19%, 9%-20%, 9%-21%, 9%-22%, 9%-23%, 9%-24%, 9%-25%, 10%-12%, 10%-14%, 10%-16%, 10%-18%, 10%-20%, 10%-22%, 10%-25%, 12%-15%, 12%-18%, 12%-21%, 12%-25%, 14%-18%, 14%-21%, 14%-23%, 14%-25%, 16%-19%, 16%-21%, 16%-23%, 16%-25%, or 25% of glycyrrhizic acid, water, an anti-microbial compound and/or a biocompatible polymer, excluding EG56, for use to treat wound healing.

Also disclosed but not part of the invention is an aqueous hydrogel comprising 2.5%-3%, 2.5%-3.5%, 2.5%-4%, 2.5%-4.5%, 2.5%-5%, 2.5%-5.5%, 2.5%-6%, 2.5%-6.5%, 2.5%-7%, 2.5%-7.5%, 2.5%-8%, 2.5%-8.5%, 2.5%-9%, 2.5%-9.5%, 2.5%-10%, 2.5%--10,5%, 2.5%-11%, 2.5%-11,5%, 2.5%-12%, 2.5%-12.5%, 2.5%-13%, 2.5%--13.5%, 2.5%-14%, 2.5%-14.5%, 2.5%-15%, 2.5%-16%, 2.5%-17%, 2.5%-18%, 2.5%-19%, 2.5%-20%, 2.5%-21%, 2.5%-22%, 2.5%-23%, 2.5%-24%, 2.5%-25%, 3%-3.5%, 3%-4%, 3%-4.5%, 3%-5%, 3%-5.5%, 3%-6%, 3%-6.5%, 3%-7%, 3%-7.5%, 3%-8%, 3%-8.5%, 3%-9%, 3%-9.5%, 3%-10%, 3%-10.5%, 3%-11%, 3%-11.5%, 3%-12%, 3%-12.5%, 3%-13%, 3%-13.5%, 3%-14%, 3%-14.5%, 3%-15%, 3%-16%, 3%-17%, 3%-18%, 3%-19%, 3%-20%, 3%-21%, 3%-22%, 3%-23%, 3%-24%, 3%-25%, 3.5%-4%, 3.5%-4.5%, 3.5%-5%, 3.5%-5.5%, 3.5%-6%, 3.5%-6.5%, 3.5%-7%, 3.5%-7.5%, 3.5%-8%, 3.5%-8.5%, 3.5%-9%, 3.5%-9.5%, 3.5%-10%, 3.5%-10.5%, 3.5%-11%, 3.5%-11.5%, 3.5%-12%, 3.5%-12.5%, 3.5%-13%, 3.5%-13.5%, 3.5%-14%, 3.5%-14.5%, 3.5%-15%, 3.5%-16%, 3.5%-17%, 3.5%-18%, 3.5%-19%, 3.5%-20%, 3.5%-21%, 3.5%-22%, 3.5%-23%, 3.5%-24%, 3.5%-25%, 4%-4.5%, 4%-5%, 4%-5.5%, 4%-6%, 4%-6.5%, 4%-7%, 4%-7.5%, 4%-8%, 4%-8.5%, 4%-9%, 4%-9.5%, 4%-10%, 4%-10.5%, 4%-11%, 4%-11.5%, 4%-12%, 4%-12.5%, 4%-13%, 4%-13.5%, 4%-14%, 4%-14.5%, 4%-15%, 4%-16%, 4%-17%, 4%-18%, 4%-19%, 4%-20%, 4%-21%, 4%-22%, 4%-23%, 4%-24%, 4%-25%, 4.5%-5%, 4.5%-5.5%, 4.5%-6%, 4.5%-6.5%, 4.5%-7%, 4.5%-7.5%, 4.5%-8%, 4.5%-8.5%, 4.5%-9%, 4.5%-9.5%, 4.5%-10%, 4.5%-10.5%, 4.5%-11%, 4.5%-11.5%, 4.5%-12%, 4.5%-12.5%, 4.5%-13%, 4.5%-13.5%, 4.5%-14%, 4.5%-14.5%, 4.5%-15%, 4.5%-16%, 4.5%-17%, 4.5%-18%, 4.5%-19%, 4.5%-20%, 4.5%-21%, 4.5%-22%, 4.5%-23%, 4.5%-24%, 4.5%-25%, 5%-5.5%, 5%-6%, 5%-6.5%, 5%-7%, 5%-7.5%, 5%-8%, 5%-8.5%, 5%-9%, 5%-9.5%, 5%-10%, 5%-10.5%, 5%-11%, 5%-11.5%, 5%-12%, 5%-12.5%, 5%-13%, 5%-13.5%, 5%-14%, 5%-14.5%, 5%-15%, 5%-16%, 5%-17%, 5%-18%, 5%-19%, 5%-20%, 5%-21%, 5%-22%, 5%-23%, 5%-24%, 5%-25%, 6%-7%, 6%-8%, 6%-9%, 6%-10%, 6%-11%, 6%-12%, 6%-13%, 6%-14%, 6%-15%, 6%-16%, 6%-17%, 6%-18%, 6%-19%, 6%-20%, 6%-21%, 6%-22%, 6%-23%, 6%-24%, 6%-25%, 7%-8%, 7%-9%, 7%-10%, 7%-11%, 7%-12%, 7%-13%, 7%-14%, 7%-15%, 7%-16%, 7%-17%, 7%-18%, 7%-19%, 7%-20%, 7%-21%, 7%-22%, 7%-23%, 7%-24%, 7%-25%, 8%-9%, 8%-10%, 8%-11%, 8%-12%, 8%-13%, 8%-14%, 8%-15%, 8%-16%, 8%-17%, 8%-18%, 8%-19%, 8%-20%, 8%-21%, 8%-22%, 8%-23%, 8%-24%, 8%-25%, 9%-10%, 9%-11%, 9%-12%, 9%-13%, 9%-14%, 9%-15%, 9%-16%, 9%-17%, 9%-18%, 9%-19%, 9%-20%, 9%-21%, 9%-22%, 9%-23%, 9%-24%, 9%-25%, 10%-12%, 10%-14%,10%-16%, 10%-18%, 10%-20%,10%-22%, 10%-25%,12%-15%, 12%-18%, 12%-21%, 12%-25%, 14%-18%, 14%-21%, 14%-23%, 14%-25%, 16%-19%, 16%-21%, 16%-23%, 16%-25%, or 25% of glycyrrhizic acid, water, an anti-microbial compound and/or a biocompatible polymer, excluding EG56, for use to treat wound healing.

Also disclosed but not part of the invention is a hydrogel for use according to claim 1, consisting of 2,5%-25% of a molecule with formula (I) as depicted below and water for use as a medicament wherein 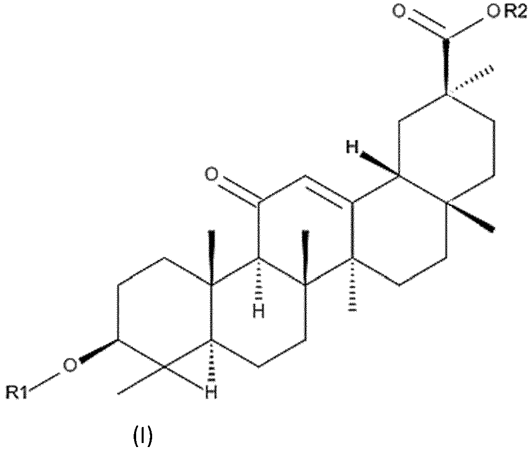
R2 is H or a C1-C20 alkyl chain,
R1 is H or a glucuronic acid moiety depicted in formula (II) 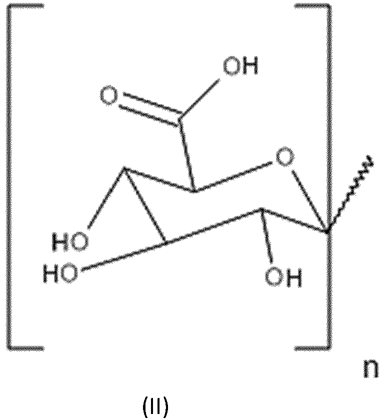
   - wherein n = 1 to 10,
   - wherein the symbol 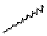 in formula (II) depicts the binding between the glucuronic acid comprising moiety and (I),
   - wherein if n = 1, the glucuronic acid moiety is a glucuronic acid monomer,
   - wherein if n > 1, the glucuronic acid comprising moiety is an alfa-1,2-linked glucuronic acid oligomer.

Also disclosed but not part of the invention is a hydrogel for use according to claim 1, consisting of 2.5%-3%, 2.5%-3.5%, 2.5%-4%, 2.5%-4.5%, 2.5%-5%, 2.5%-5.5%, 2.5%-6%, 2.5%-6.5%, 2.5%-7%, 2.5%-7.5%, 2.5%-8%, 2.5%-8.5%, 2.5%-9%, 2.5%-9.5%, 2.5%-10%, 2.5%--10,5%, 2.5%-11%, 2.5%-11,5%, 2.5%-12%, 2.5%-12.5%, 2.5%-13%, 2.5%--13.5%, 2.5%-14%, 2.5%-14.5%, 2.5%-15%, 2.5%-16%, 2.5%-17%, 2.5%-18%, 2.5%-19%, 2.5%-20%, 2.5%-21%, 2.5%-22%, 2.5%-23%, 2.5%-24%, 2.5%-25%, 3%-3.5%, 3%-4%, 3%-4.5%, 3%-5%, 3%-5.5%, 3%-6%, 3%-6.5%, 3%-7%, 3%-7.5%, 3%-8%, 3%-8.5%, 3%-9%, 3%-9.5%, 3%-10%, 3%-10.5%, 3%-11%, 3%-11.5%, 3%-12%, 3%-12.5%, 3%-13%, 3%-13.5%, 3%-14%, 3%-14.5%, 3%-15%, 3%-16%, 3%-17%, 3%-18%, 3%-19%, 3%-20%, 3%-21%, 3%-22%, 3%-23%, 3%-24%, 3%-25%, 3.5%-4%, 3.5%-4.5%, 3.5%-5%, 3.5%-5.5%, 3.5%-6%, 3.5%-6.5%, 3.5%-7%, 3.5%-7.5%, 3.5%-8%, 3.5%-8.5%, 3.5%-9%, 3.5%-9.5%, 3.5%-10%, 3.5%-10.5%, 3.5%-11%, 3.5%-11.5%, 3.5%-12%, 3.5%-12.5%, 3.5%-13%, 3.5%-13.5%, 3.5%-14%, 3.5%-14.5%, 3.5%-15%, 3.5%-16%, 3.5%-17%, 3.5%-18%, 3.5%-19%, 3.5%-20%, 3.5%-21%, 3.5%-22%, 3.5%-23%, 3.5%-24%, 3.5%-25%, 4%-4.5%, 4%-5%, 4%-5.5%, 4%-6%, 4%-6.5%, 4%-7%, 4%-7.5%, 4%-8%, 4%-8.5%, 4%-9%, 4%-9.5%, 4%-10%, 4%-10.5%, 4%-11%, 4%-11.5%, 4%-12%, 4%-12.5%, 4%-13%, 4%-13.5%, 4%-14%, 4%-14.5%, 4%-15%, 4%-16%, 4%-17%, 4%-18%, 4%-19%, 4%-20%, 4%-21%, 4%-22%, 4%-23%, 4%-24%, 4%-25%, 4.5%-5%, 4.5%-5.5%, 4.5%-6%, 4.5%-6.5%, 4.5%-7%, 4.5%-7.5%, 4.5%-8%, 4.5%-8.5%, 4.5%-9%, 4.5%-9.5%, 4.5%-10%, 4.5%-10.5%, 4.5%-11%, 4.5%-11.5%, 4.5%-12%, 4.5%-12.5%, 4.5%-13%, 4.5%-13.5%, 4.5%-14%, 4.5%-14.5%, 4.5%-15%, 4.5%-16%, 4.5%-17%, 4.5%-18%, 4.5%-19%, 4.5%-20%, 4.5%-21%, 4.5%-22%, 4.5%-23%, 4.5%-24%, 4.5%-25%, 5%-5.5%, 5%-6%, 5%-6.5%, 5%-7%, 5%-7.5%, 5%-8%, 5%-8.5%, 5%-9%, 5%-9.5%, 5%-10%, 5%-10.5%, 5%-11%, 5%-11.5%, 5%-12%, 5%-12.5%, 5%-13%, 5%-13.5%, 5%-14%, 5%-14.5%, 5%-15%, 5%-16%, 5%-17%, 5%-18%, 5%-19%, 5%-20%, 5%-21%, 5%-22%, 5%-23%, 5%-24%, 5%-25%, 6%-7%, 6%-8%, 6%-9%, 6%-10%, 6%-11%, 6%-12%, 6%-13%, 6%-14%, 6%-15%, 6%-16%, 6%-17%, 6%-18%, 6%-19%, 6%-20%, 6%-21%, 6%-22%, 6%-23%, 6%-24%, 6%-25%, 7%-8%, 7%-9%, 7%-10%, 7%-11%, 7%-12%, 7%-13%, 7%-14%, 7%-15%, 7%-16%, 7%-17%, 7%-18%, 7%-19%, 7%-20%, 7%-21%, 7%-22%, 7%-23%, 7%-24%, 7%-25%, 8%-9%, 8%-10%, 8%-11%, 8%-12%, 8%-13%, 8%-14%, 8%-15%, 8%-16%, 8%-17%, 8%-18%, 8%-19%, 8%-20%, 8%-21%, 8%-22%, 8%-23%, 8%-24%, 8%-25%, 9%-10%, 9%-11%, 9%-12%, 9%-13%, 9%-14%, 9%-15%, 9%-16%, 9%-17%, 9%-18%, 9%-19%, 9%-20%, 9%-21%, 9%-22%, 9%-23%, 9%-24%, 9%-25%, 10%-12%, 10%-14%, 10%-16%, 10%-18%, 10%-20%, 10%-22%, 10%-25%, 12%-15%, 12%-18%, 12%-21%, 12%-25%, 14%-18%, 14%-21%, 14%-23%, 14%-25%, 16%-19%, 16%-21%, 16%-23%, 16%-25%, or 25% of a molecule with formula (I) as depicted below and water for use as a medicament wherein 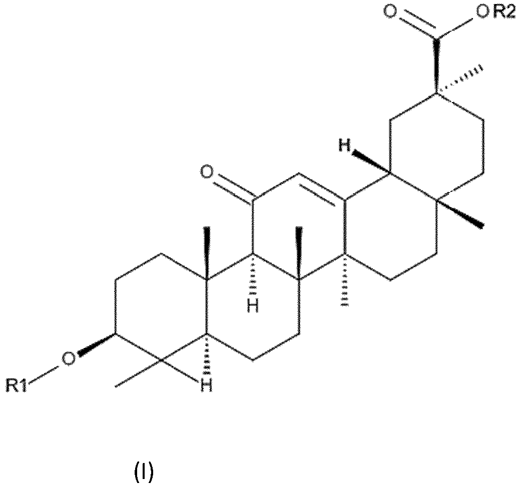
R2 is H or a C1-C20 alkyl chain,
R1 is H or a glucuronic acid moiety depicted in formula (II) 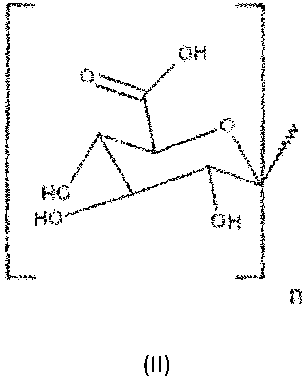
   - wherein n = 1 to 10,
   - wherein the symbol 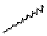 in formula (II) depicts the binding between the glucuronic acid comprising moiety and (I),
   - wherein if n = 1, the glucuronic acid moiety is a glucuronic acid monomer,
   - wherein if n > 1, the glucuronic acid comprising moiety is an alfa-1,2-linked glucuronic acid oligomer.

Also disclosed but not part of the invention is a molecule with formula (I) as depicted below for use to treat wound healing wherein 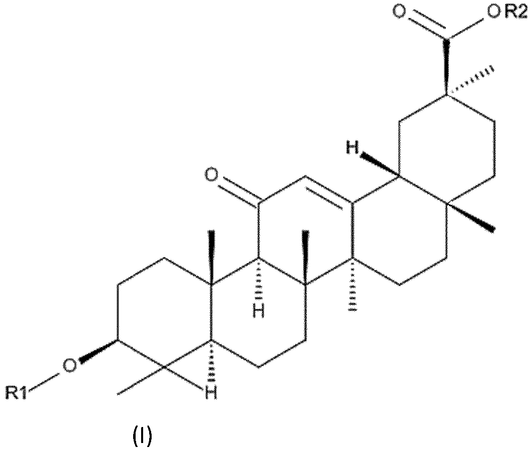
R2 is H or a C1-C20 alkyl chain,
R1 is H or a glucuronic acid moiety depicted in formula (II) 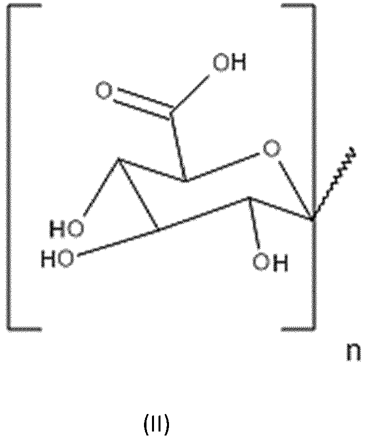
   - wherein n = 1 to 10,
   - wherein the symbol 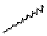 in formula (II) depicts the binding between the glucuronic acid comprising moiety and (I),
   - wherein if n = 1, the glucuronic acid moiety is a glucuronic acid monomer,
   - wherein if n > 1, the glucuronic acid comprising moiety is an alfa-1,2-linked glucuronic acid oligomer.

The term "C1-C20-alkyl" refers to a linear or branched-chain saturated, mono- unsaturated and poly-unsaturated hydrocarbyl substituent (i.e., a substituent obtained from a hydrocarbon by removal of a hydrogen). Mono- and poly-unsaturated substituents, also called alkenyl, have 2 to 20 carbon atoms. The alkenyl group may exist as the pure E *(entgegen)* form, the pure Z *(zusammen)* form, or any mixture thereof. Poly-unsaturated includes multiple double bonds and one or more triple bonds. Such triple bond containing alkyl groups, a so called alkynyl group, has 2 to 20 carbon atoms. Furthermore, where the compounds of the invention carry an acidic moiety (e.g. in case wherein R2 = H in formula (I)), suitable pharmaceutically acceptable salts thereof may include alkali metal salts, e.g. sodium or potassium salts; alkaline earth metal salts, e.g. calcium or magnesium salts; and salts formed with suitable organic ligands, e.g. quaternary ammonium salts. In another embodiment, base salts are formed from bases which form nontoxic salts, including aluminum, arginine, benzathine, choline, diethylamine, diolamine, glycine, lysine, meglumine, olamine, tromethamine and zinc salts. In one embodiment, hemisalts of acids and bases may also be formed, for example, hemisulphate and hemicalcium salts.

The present invention also includes isotopically labelled compounds, which are identical to those recited in formula (I), but for the fact that one or more atoms are replaced by an atom having an atomic mass or mass number different from the atomic mass or mass number usually found in nature. Examples of isotopes that may be incorporated into compounds of the present invention include isotopes of hydrogen, carbon, nitrogen, oxygen, phosphorous, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. Compounds of the present invention and pharmaceutically acceptable salts of said compounds or which contain the aforementioned isotopes and/or other isotopes of other atoms are within the scope of this invention. Certain isotopically labeled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, may afford certain therapeutic advantages resulting from greater metabolic stability, for example increased in vivo half-life or reduced dosage requirements and, hence, may be preferred in some circumstances. Isotopically labelled compounds of formula I of this invention may generally be prepared by substituting a readily available isotopically labelled reagent for a non-isotopically labelled reagent.

In another specific embodiment the invention provides a wound healing composition for topical application to an external wound of a mammal, said composition comprising a hydrogel as defined in the claims.

In yet another embodiment the invention provides a topical plaster according to claim 4 (or an adhesive bandage or a band-aid which are equivalent terms) comprising a hydrogel consisting of glycyrrhizic acid at 3,5%-25% by weight of the molecule dissolved in water.

In yet another embodiment the invention provides a topical plaster according to claim 4 (or an adhesive bandage or a band-aid which are equivalent terms) comprising a hydrogel consisting of glycyrrhizin, at 3,5%-25% by weight of the molecule dissolved in water.

In yet another embodiment the invention provides a topical plaster according to claim 4 (or an adhesive bandage or a band-aid which are equivalent terms) comprising a hydrogel consisting of glycyrrhizin, at 3,5%-20% by weight of the molecule dissolved in water.

In yet another embodiment the invention provides a topical plaster according to claim 4 (or an adhesive bandage or a band-aid which are equivalent terms) comprising a hydrogel consisting of glycyrrhizin, at 3,5%-15% by weight of the molecule dissolved in water.

In yet another embodiment the invention provides a topical plaster according to claim 4 (or an adhesive bandage or a band-aid which are equivalent terms) comprising a hydrogel consisting of glycyrrhizin, at 3.5%-10% by weight of the molecule dissolved in water.

In yet another embodiment the invention provides a topical plaster according to claim 4 (or an adhesive bandage or a band-aid which are equivalent terms) comprising a hydrogel consisting of glycyrrhizin, at 5%-15% by weight of the molecule dissolved in water.

In yet another embodiment the invention provides a topical plaster according to claim 4 (or an adhesive bandage or a band-aid which are equivalent terms) comprising a hydrogel consisting of glycyrrhizin, at 5%-10% by weight of the molecule dissolved in water.

In yet another embodiment the invention provides a topical plaster according to claim 4 (or an adhesive bandage or a band-aid which are equivalent terms) comprising a hydrogel essentially consisting of glycyrrhizin, at 3.5%-25% by weight of the molecule dissolved in water.

In yet another embodiment the invention provides a topical plaster according to claim 4 (or an adhesive bandage or a band-aid which are equivalent terms) comprising a hydrogel essentially consisting of glycyrrhizin, at 3.5%-20% by weight of the molecule dissolved in water.

In yet another embodiment the invention provides a topical plaster according to claim 4 (or an adhesive bandage or a band-aid which are equivalent terms) comprising a hydrogel essentially consisting of glycyrrhizin, at 3.5%-15% by weight of the molecule dissolved in water.

In yet another embodiment the invention provides a topical plaster according to claim 4 (or an adhesive bandage or a band-aid which are equivalent terms) comprising a hydrogel essentially consisting of glycyrrhizin, at 3.5%-10% by weight of the molecule dissolved in water.

In yet another embodiment the invention provides a topical plaster according to claim 4 (or an adhesive bandage or a band-aid which are equivalent terms) comprising a hydrogel essentially consisting of glycyrrhizin, at 5%-15% by weight of the molecule dissolved in water.

In yet another embodiment the invention provides a topical plaster according to claim 4 (or an adhesive bandage or a band-aid which are equivalent terms) comprising a hydrogel essentially consisting of glycyrrhizin, at 5%-10% by weight of the molecule dissolved in water.

Mammals include humans, pets (e.g. dogs and cats), veterinary animals (e.g. horses, pigs, cows), livestock and research animals.

As used herein, the term "wound" includes an injury to any tissue, including, for example, acute, delayed, or difficult to heal wounds, and chronic wounds. Examples of wounds may include both open and closed wounds. Wounds include, for example, burns, incisions, excisions, lacerations, abrasions, puncture on penetrating wounds, surgical wounds, contusions, hematoma, crushing injuries, and ulcers. Also included are wounds that do not heal at expected rates, as is expected in diabetic patients. The term "wound" may also include for example, injuries to the skin and subcutaneous tissue initiated in different ways (e.g. pressure sores from extended bed rest and wounds induced by trauma) and with varying characteristics. Wounds may be classified into one of four grades depending on the depth of the wound: i) Grade I: wounds limited to the epithelium; ii) Grade II: wounds extending into the dermis; iii) Grade III: wounds extending into the subcutaneous tissue; and iv) Grade IV (or full-thickness wounds): wounds wherein bones are exposed (e.g. a bony pressure point such as the greater trochanter or the sacrum).

The term "partial thickness wound" refers to wounds that encompass Grades I-III; examples of partial thickness wounds include pressure sores, venous stasis ulcers, and diabetic ulcers. The present invention contemplates treating all wounds of a type that do not heal at expected rates, including, delayed-healing wounds, incompletely healing wounds, and chronic wounds.

By "wound that does not heal at an/the expected rate" is meant an injury to any tissue that does not heal in an expected or typical time frame, including delayed or difficult to heal wounds (including delayed or incompletely healing wounds), and chronic wounds. Examples of wounds that do not heal at the expected rate include ulcers such as diabetic ulcers, vasculitic ulcers, arterial ulcers, venous ulcers, venous stasis ulcers, burn ulcers, infectious ulcers, trauma-induced ulcers, pressure ulcers, decubitus ulcers, ulcerations associated with pyoderma gangrenosum, and mixed ulcers. Other wounds that do not heal at expected rates include dehiscent wounds.

As used herein, a delayed or difficult to heal wound may include, for example, a wound that is characterized at least in part by 1) a prolonged inflammatory phase, 2) a slow forming extracellular matrix, and/or 3) a decreased rate of epithelialization or closure.

The term "chronic wound" refers to a wound that has not healed. Wounds that do not heal within 6 weeks, for example, are considered chronic. Chronic wounds include, for example, pressure ulcers, decubitus ulcers, diabetic ulcers including diabetic foot and leg ulcers, slow or non-healing venous ulcers, venous stasis ulcers, arterial ulcers, vasculitic ulcers, burn ulcers, trauma-induced ulcers, infectious ulcers, mixed ulcers, and pyoderma gangrenosum. The chronic wound may be an arterial ulcer that comprises ulcerations resulting from complete or partial arterial blockage. The chronic wound may be a venous or venous stasis ulcer that comprises ulcerations resulting from a malfunction of the venous valve and the associated vascular disease.

As used herein, the term "dehiscent wound" refers to a wound, usually a surgical wound, which has ruptured or split open. In certain embodiments, a method of treating a wound that does not heal at the expected rate is provided wherein the wound is characterized by dehiscence.

In addition to the definition previously provided, the term "wound" may also include for example, injuries to the skin and subcutaneous tissue initiated in different ways (e.g., pressure sores from extended bed rest and wounds induced by trauma) and with varying characteristics.

The following examples are provided to better illustrate particular embodiments, and they should not be considered limiting the application. The application is limited by the claims.

### Examples

### 1.Gelation experiments with glycyrrhizin

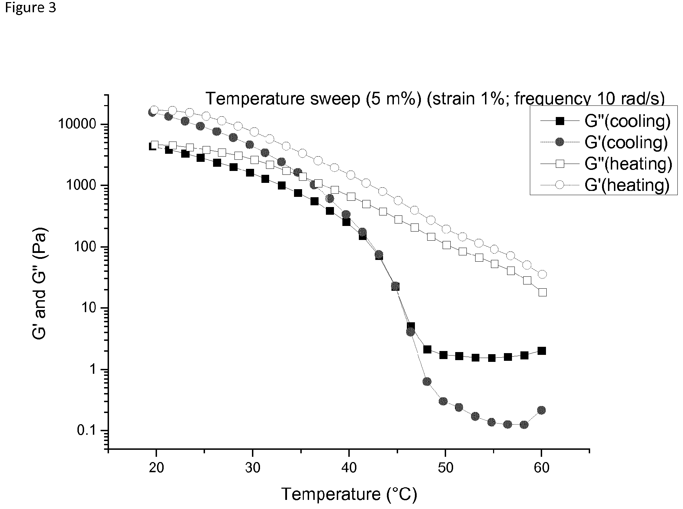

Different hydrogels were prepared by varying the glycyrrhizin concentrations (abbreviated herein as Gly) dissolved in water as described in detail in the materials and methods section. One hydrogel contained 2.5% Gly (*id est* 2.5 gram Gly and water is added until 100 gram of water) and another hydrogel contained 5% Gly (*id est* 5 gram Gly and water is added until 100 gram of water). Subsequently several rheological test were conducted with these two hydrogels to quantify the properties of the hydrogels, the first test determined the gelation/degelation point, the second test determined the strength and the third test is a stress sweep. The first rheological test includes a temperature sweep wherein the G' (elastic modulus) and G" (storage modulus) are measured in function of the temperature. This gelation temperature is determined when both curves cross. It was observed that when the liquid sample of 2.5% Gly is cooled down the values for G' and G" raise (see Figure 2), this indicates that the sample first behaves as a liquid and by cooling a hydrogel is formed. On the contrary, the temperature sweep at higher temperatures for the 2.5% Gly sample shows low values for both G' and G" indicating that the sample is liquid. Thus specifically for the 2.5% Gly sample by decreasing the temperature both G' and G" values increase and at 40°C, G' becomes larger than G" which indicates that 40°C is the gelation point for the 2.5% Gly sample. When this hydrogel is cooled a hysteresis appears and the G' remains larger than G". Similar conclusions can be drawn for the 5% Gly sample (see Figure 3), here the gelation temperature is higher and was determined to be at 45°C. We conclude that at body temperature hydrogels are formed for both the 2.5% Gly and the 5% Gly samples. No strains and frequency sweeps were done at 60°C as both samples acted as a liquid at this temperature and the obtained values are meaningless.

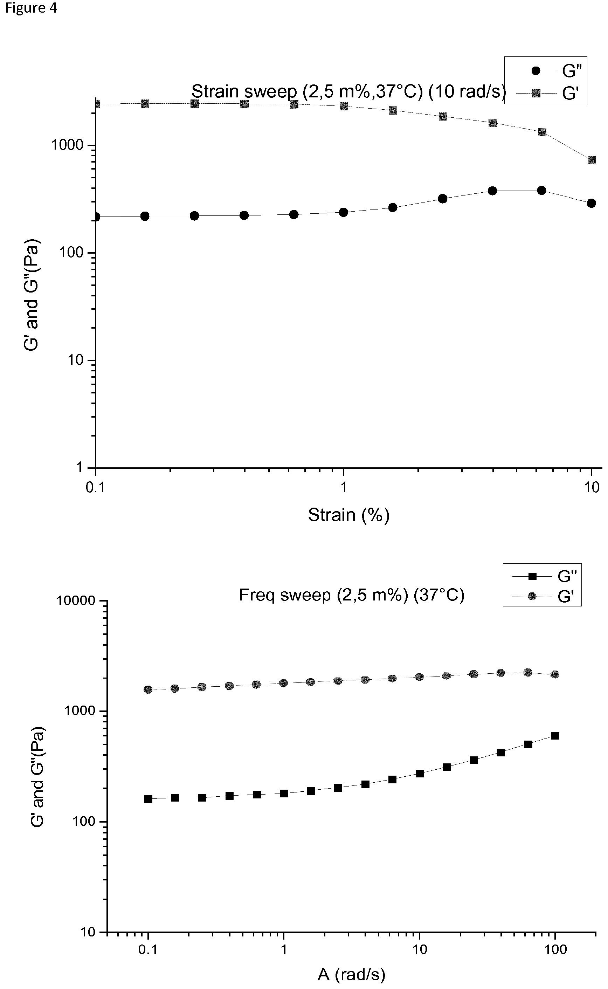

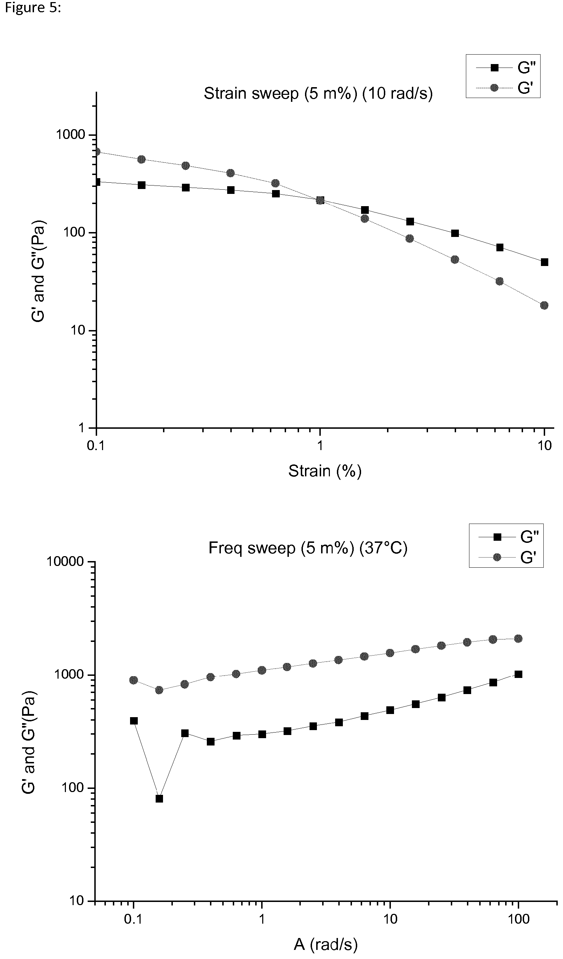

The following rheological measurements at 37°C monitor how the hydrogel acts at body temperature, being the application temperature for topical applications to use in mammals such as humans. These test gives us insight in the strength of the hydrogel. The strain sweep is a good indication for the linearity of the measurement. The hydrogel containing 2.5% Gly has a G' value above 1000 Pa (see Figure 4). Similar values are obtained for the 5% Gly hydrogel however the values for G' are lower providing slightly less rigid gels (see Figure 5). Due to this decreased value the difference between both G' and G" is decreased for the 5 % Gly sample. These tests show that strong hydrogels are formed at body temperature.

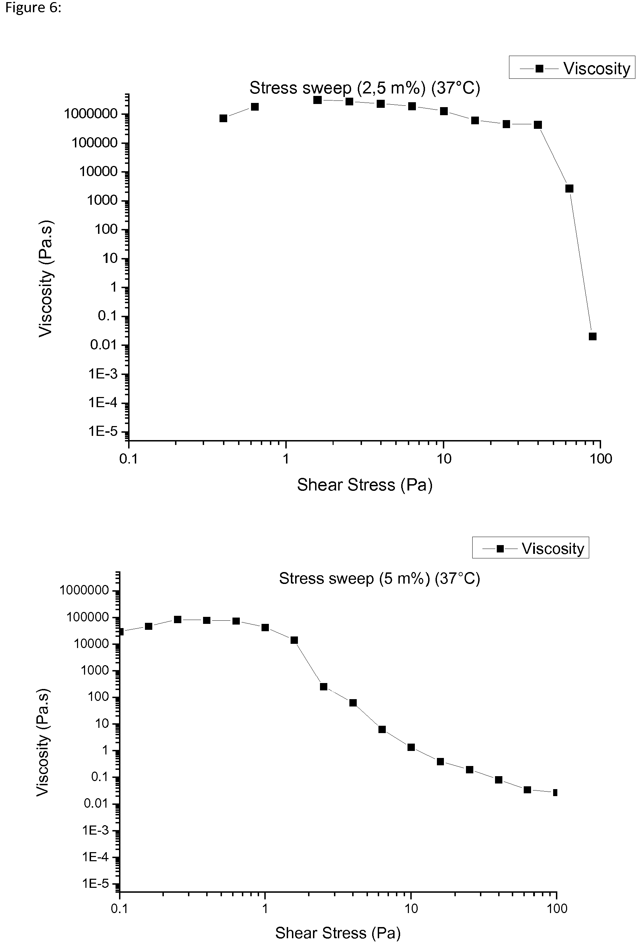

The next rheological measurements investigate how the gels behaves under stress. This indicates how the gel behaves when it is injected by a syringe. This stress sweep is measured by plotting the viscosity in function of stress. The shear stress is slightly different in both samples: the hydrogel consisting of 2.5% Gly deforms less at higher shear stress and suddenly flows, this is proven by the sudden drop in viscosity (100000 to 0.01 Pa.s). The hydrogel consisting of 5% Gly gradually decreases in viscosity (see Figure 6). This gel is less viscous than the 2.5% Gly hydrogel These experiments shows the possibility to inject both hydrogel compositions.

### 2. Gelation experiments with a broad range of glycyrrhizin concentrations

In a more systematic approach a wider range of hydrogels with different glycyrrhizin (Gly) concentrations was prepared for rheological measurements. Thereto appropriate amounts of Gly were dissolved in hot deionized water at 80°C while stirring. Eight (8) different solutions with specific concentrations were prepared as shown in the table below. The vessels were removed from the heating block and cooled in a refrigerator at 4°C. The solution formed gels as the temperature of the solution decreased from 80°C.

**Table 1: preparation of 8 different glycyrrhizin (Gly) concentrations ranging from 2% to 25% of glycyrrhizin.**

| m% glycyrrhizin (Gly) | g/20 ml |
|---|---|
| 25 | 5 |
| 20 | 4 |
| 13 | 2.6 |
| 9 | 1.8 |
| 5.6 | 1.12 |
| 2.5 | 0.5 |
| 2 | 0.4 |

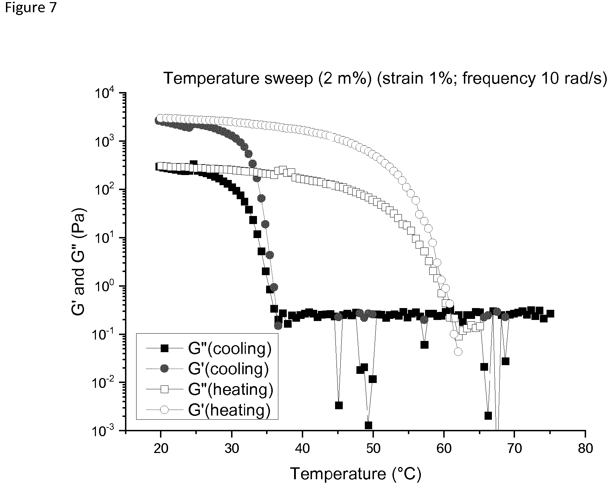

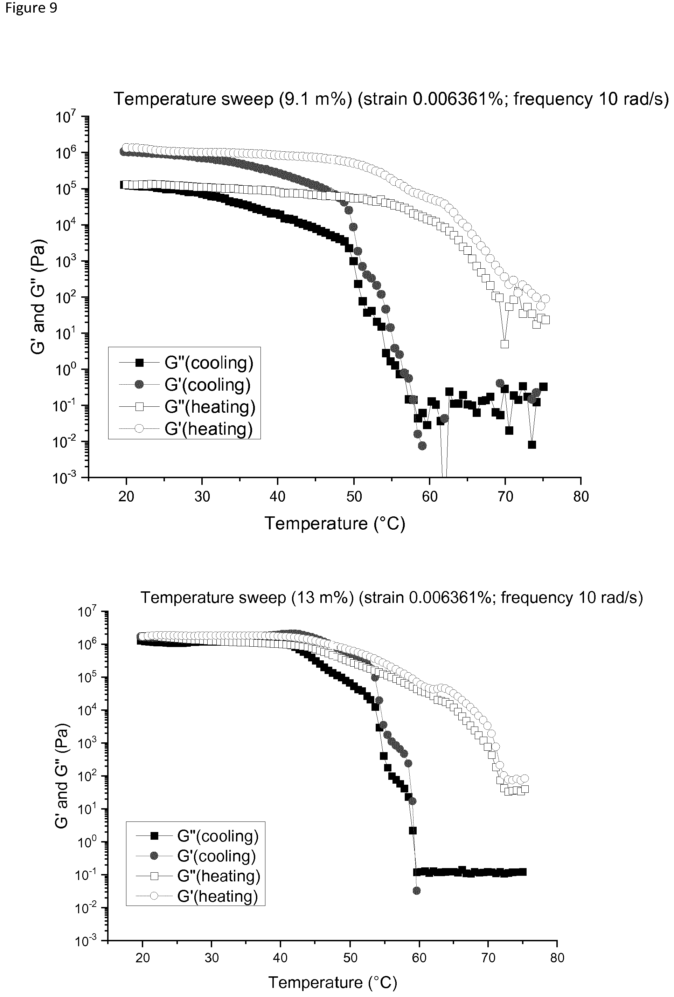

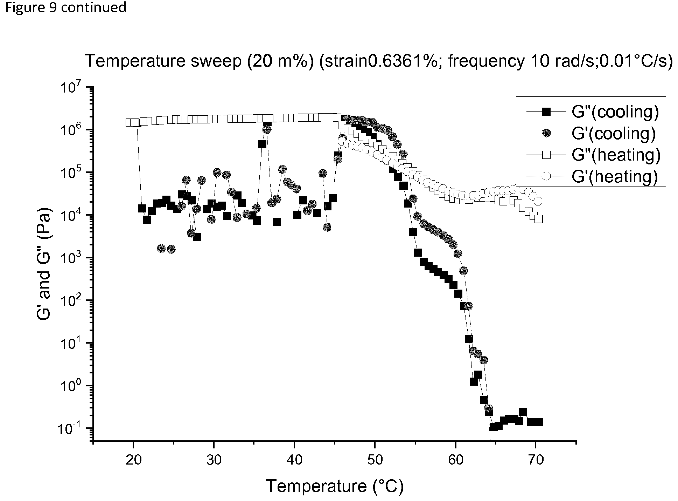

Rheological measurements on the different solutions were carried out on an Anton Paar Rheometer MCR 501a - as described in Example 1 and in the materials and methods section - with a normal bob and couette setup. The gel samples were melted at 75°C, this improves their handling and the transfer into the couette. The liquid sample was transferred into the couette. The temperature sweeps in the figures 7, 8 and 9 show the value of G' and G" in function of the temperature. Low values of G' and G" indicate a liquid sample and minor structure formation. When these values increase there is an indication of structure formation and the sample becomes solid. For G'>G" the elastic (solid) portion of the behaviour is dominant and the hydrogel has a more solid-like behavior. The sample was stabilized for 30 min to ensure a homogenous temperature in the sample. The cooling rate was 0.01°C/s. These measurements indicate whether the sample exists as a hydrogel at different temperatures, particularly at 37°C. After cooling, the sample was heated starting from 20°C until 75°C or 60° depending on the sample with a heating rate of 0.01°C/s. We included the values of G' and G" of the cooling curve because as this gives a better idea from when the gel is formed. Because in the heating curve the gel is already formed, less heat is conducted in the sample and may therefore slower down the melting and breakdown of the hydrogel. When the G'>G" the sample has more solid propterties and a hydrogel is formed, this is the rheological definition of a hydrogel.

The first example shown in Figure 7 is the 2 m% Gly hydrogel. Figure 7 shows that at 37°C, both G' and G" have values below 1 indicating a low viscosity and the non-existence of a hydrogel at 37°C. G'<G" indicating no gel is formed.

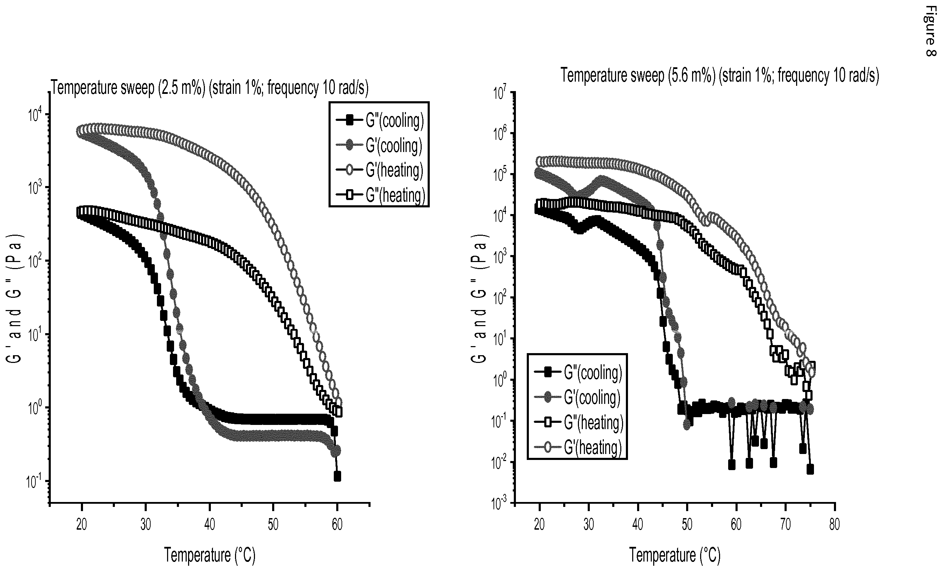

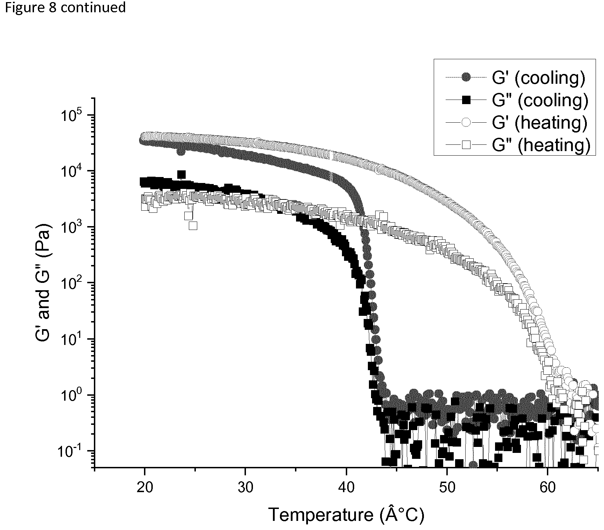

Starting from 2.5m% (see Figure 8, left) the value for G' and G" at 37°C are 216 Pa and 3510 Pa respectively during the heating cycle (G'>G" at 38°C). The 5.6m% sample values for G' and G" at 37°C are 1.62*10⁵ Pa and 1.48*10⁴ Pa, (G'>G" at 49.3°C) These values indicate the formation of a strong hydrogel.

Table 2 shows the values for both G' and G" at 37°C for different concentrations of Gly, this shows the strength of the hydrogel at this given temperature and the practical use. It is clear that the values obtained from 2.5m% are higher and a large difference between both is observed. Important is also the last column which indicates the turning point when G'>G" - the latter is the rheological definition of a hydrogel (see J.-M. Guenet (2016) Organogels, Thermodynamics, Structure, Solvent Role, and Properties ISBN: 978-3-319-33176-8). At body temperature the solid part becomes more dominant and thus we can conclude that a hydrogel is formed at body temperature

**Table 2: G' and G"" values for a range of glycyrrhizin concentrations**

| Concentration of Gly (m%) | G' | G" | G'>G" |
|---|---|---|---|
| 2 | 1830 | 257 | 36°C |
| 2.5 | 3510 | 216 | 38.3°C |
| 5.6 | 1.62*10⁵ | 1.48*10⁴ | 49.3°C |
| 9.1 | 9.19*10⁵ | 9.26*10⁴ | 56.6°C |
| 13 | 1.76*10⁶ | 1.2*10⁶ | 59°C |
| 20 | 1.88*10⁶ | not determined | 63.5°C |

### 3. Wound healing experiments in mice

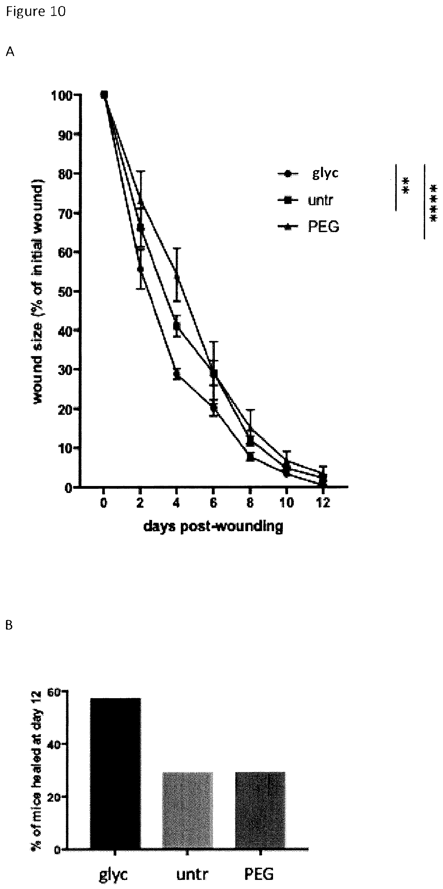

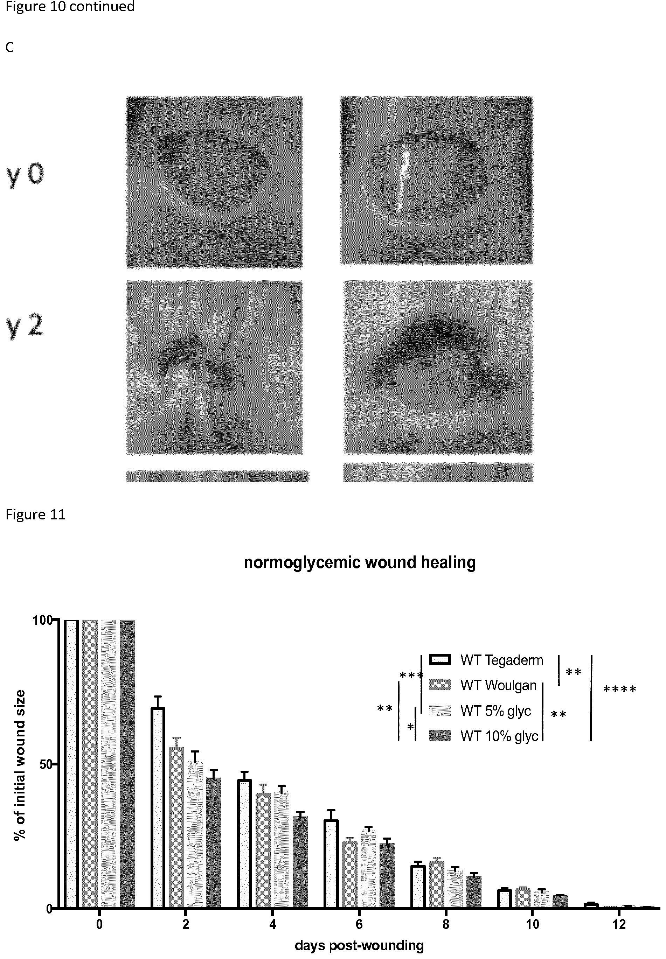

In this experiment we investigated whether the hydrogels of the invention are capable to influence the healing dynamics of wounds induced in mice. Thereto an 8 mm full-thickness skin wound is inflicted onto the shaved back skin of the anaesthetized mice. Three different experimental topical treatments were chosen : i) in the first condition, the mice were treated with 0.5 ml of hydrogel containing 5% Gly, ii) in the second setup 0.5 ml of a composition consisting of 5% Gly and the polymer EG56 and iii) in the third condition, the mice were left untreated. Newly-inflicted wounds were treated with previously described treatments at the time of wounding. At day 2, 4, 6, 8 and 10 post-wounding, mice were treated with 0,5 ml of glycyrrhizin hydrogel, EG56 hydrogel or left untreated. Wound healing was measured every other day until wound closure by means of electronic calipers. It was clear that the wound size - after several days - for the mice treated with 5% Gly hydrogel was significant lower compared to mice treated with the EG56/5% Gly composition or compared to untreated mice. Specifically, at day 4 (see Figure 10) the wound size is 30% of the initial wound for the 5% Gly hydrogel while the untreated wounds show a wound size of more than 40% of the initial wound size and the EG56/5%Gly treated wounds exhibit a wound size of more than 50% of the initial wound size. It was also noted that the EG56/5% Gly composition caused irritation of the skin which induced an exacerbated inflammatory response and delayed injury repair. After 12 days post-wounding 58% of the mice receiving the hydrogel of 5% Gly showed complete closure of the wound, while in the other two setups (untreated and EG56/5%Gly composition) only 30% of mice showed closed wounds.

### 4.Wound healing in a larger cohort of mice - in normoglycemic and diabetic conditions

50 normoglycemic (wild type C57BI/6 mice (WT) and 50 diabetic female mice (LepR^{db/db} mice model described in Guilbaud A. et al (2019) Diabetes Metab. Res. Rev. 35(2): e3103) of 7 weeks old were wounded with an 8 mm circular biopsy needle to create one full-thickness skin wound on the back skin of each mouse.

Then, mice were treated with 500µl of 4 different hydrogels starting at the day of wounding and repeatedly applied on day 2, 4, 6, 8, 10 and 12 post-wounding

The 4 different hydrogels used were:
1) 3M^{™} Tegaderm^{™} hydrogel wound filler (the standard-of-care hydrogel)
2) Woulgan^{®} (a bio-active beta-glucan) hydrogel for wound healing
3) 5% glycyrrhizin hydrogel
4) 10% glycyrrhizin hydrogel

Wound sizes were measured every other day post-wounding and mice were sacrificed at day 14 post-wounding

Wound closure for wild type mice (normoglycemic conditions) over time is shown in Figure 11.

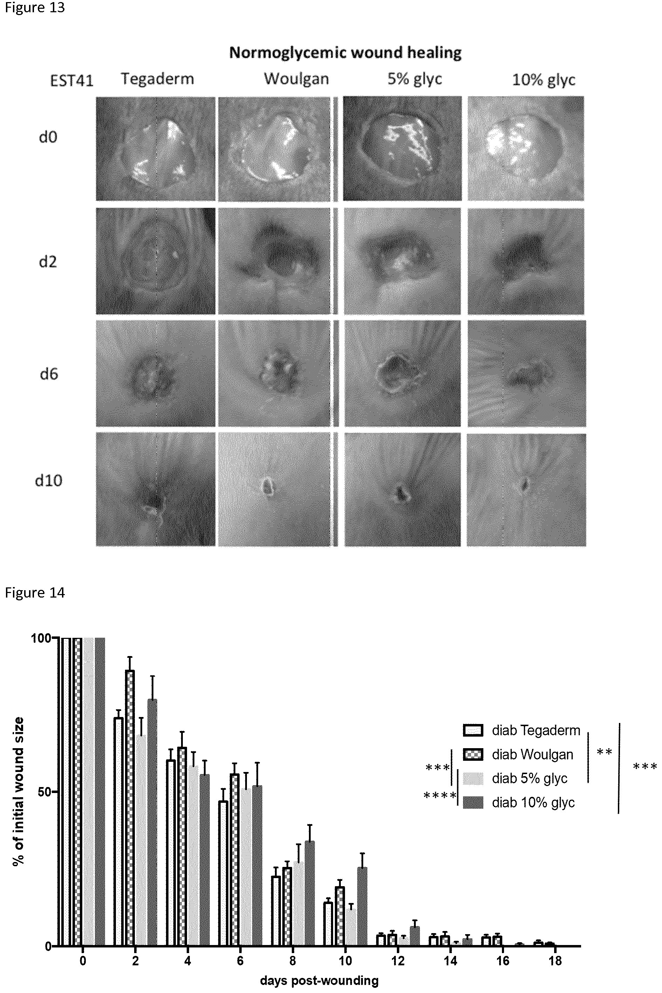

Figure 13 shows pictures of wounds induced in normoglycemic mice and treated with the 4 different hydrogels.

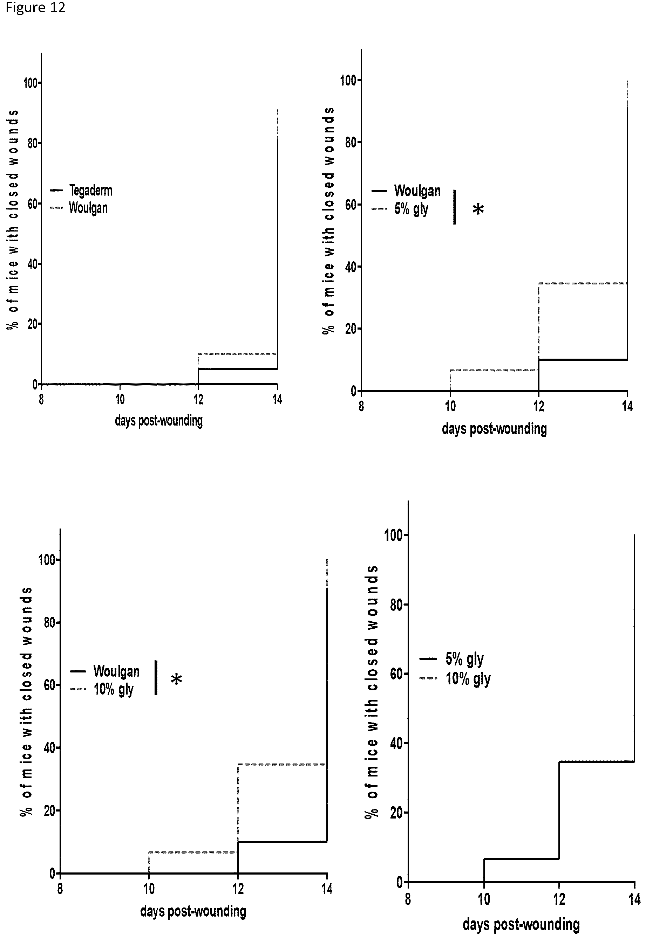

In addition, the percentage (%) of mice with healed wounds at different time-points is shown in Figure 12.

Based on the data depicted in Figures 11, 12 and 13 we conclude that a hydrogel containing 10% glycyrrhizin outperforms all other hydrogels on healing rate of normoglycemic wounds over time.

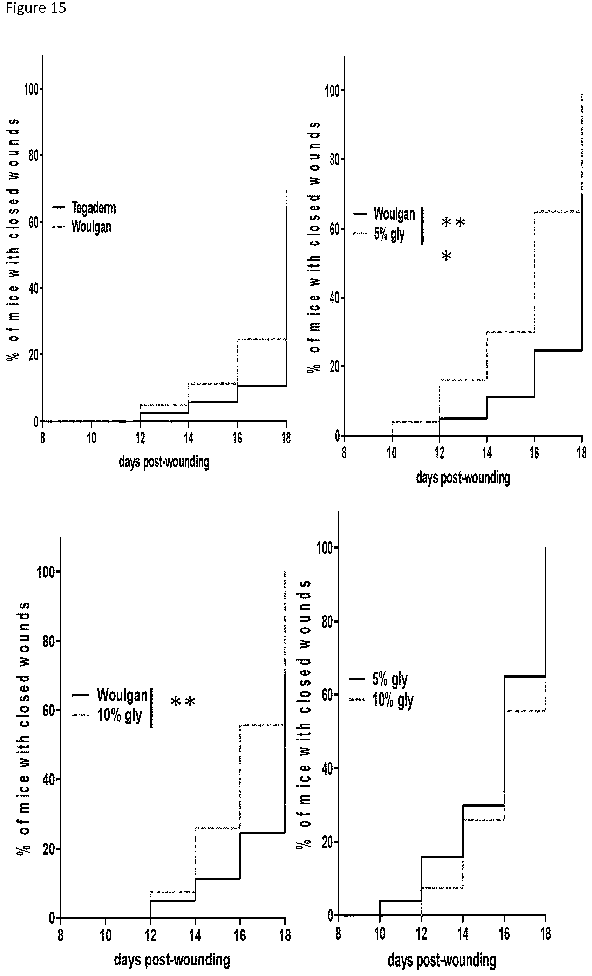

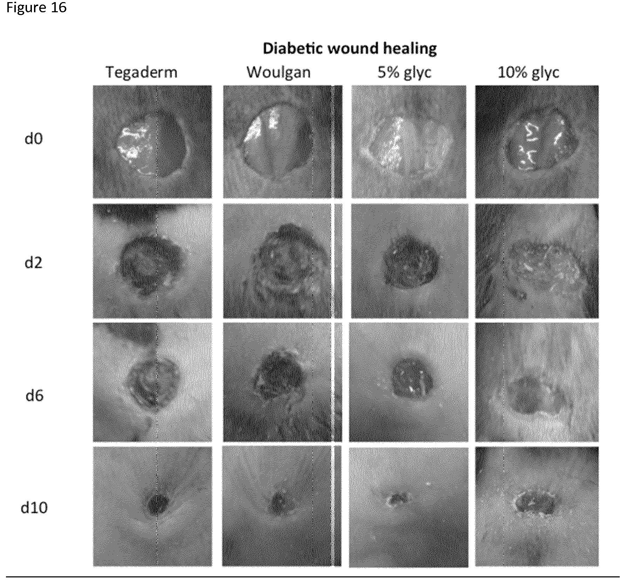

The wound closure over time for diabetic mice (mice with a LepR^{db/db} genetic background) is shown in Figures 14, 15 and 16.

### 5.Therapeutic potential of glycyrrhizin hydrogel formulations in pigs

4 male pigs aged 20 weeks were wounded (1,5 cm x 1,5 cm full-thickness) on the back skin. Wounds were treated every other day (day 0, 2, 4, 6, 8, 10 and 12 post-wounding) with 3 different hydrogels:
(1) untreated (2 ml per treatment)
(2) Tegaderm^{™} wound filler (3M) (2 ml per treatment)
(3) 5% Glycyrrhyzin hydrogel (2 ml per treatment)

Wounds were placed 5 cm apart from each other under general and local anesthesia. A Tegaderm^{™} adhesive dressing was added on wounds after treatments. Pigs were sacrificed at day 14 post-wounding.

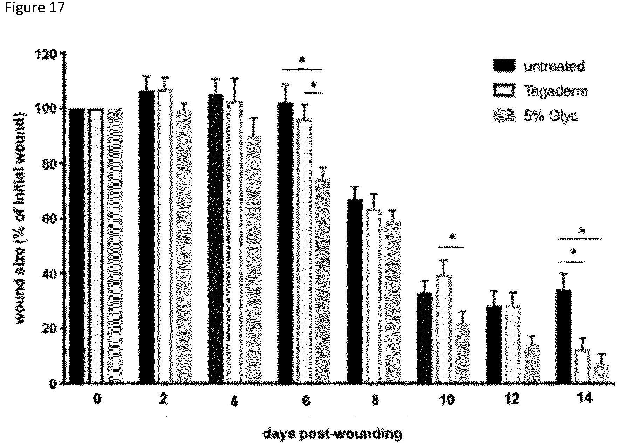

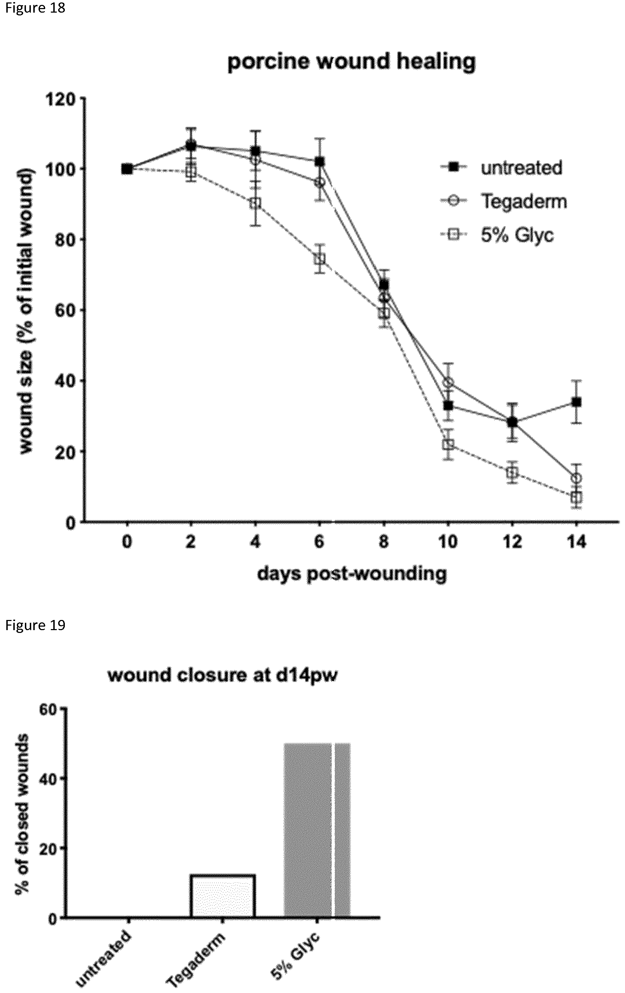

Wound closure over time is depicted in Figures 17 and 18.

Figures 17 and 18 show the rate of wound healing of porcine skin treated with different hydrogels or left untreated. 6 full-thickness skin wounds (1,5 cm x 1,5 cm) were inflicted on porcine skin (n=4). Wounds were treated every other day with 2ml Tegaderm^{™} wound filler (tegaderm), 2ml 5% Glycyrrhizin hydrogel (5% Glyc) or left untreated. Wounds were measured at the indicated time-points, histograms represent mean ± SEM (Two-way ANOVA; * 0.01<P<0.05; ** 0.001<P<0,01; *** 0.0001<P<0.001).

It is apparent from Figures 17 and 18 that treatment of the wounds with the hydrogel containing 5% Glycyrrhizin results in faster wound healing rates than observed in wounds treated with Tegaderm hydrogel or left untreated.

At day 14 post-wounding 15% of porcine wounds treated with Tegaderm wound filler (n=8) were healed versus 50% of wounds treated with 5% Glycyrrhizin and none of the wounds healed that were left untreated (see Figure 19).

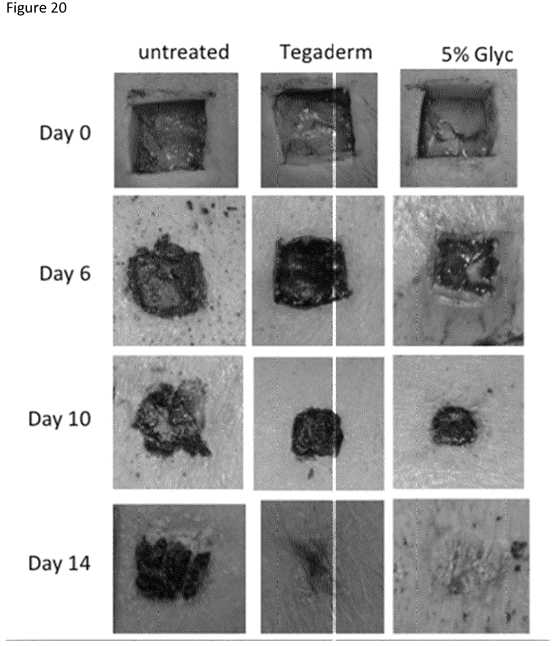

Figure 20 depicts pictures of cutaneous pig wounds treated with the different hydrogels and the effects on specific days of treatment.

### 6. Analysis of efficacy of hydrogels consisting of derivatives of glycyrrhyzine on cutaneous wound healing in mice (reference example)

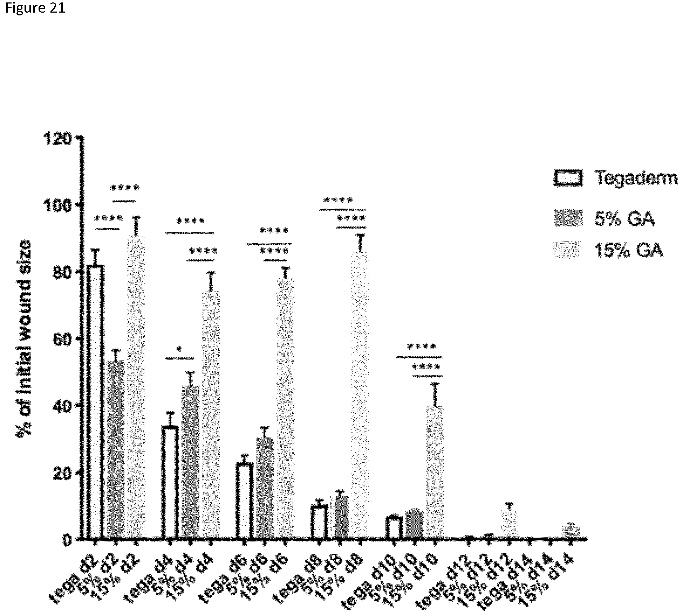

In a next step we evaluate variants of formula (I) for their effect on wound healing. Specifically, glycyrrhetinic acid (wherein in formula (I) the R1 and R2 groups are hydrogen (H)) was evaluated. The latter molecule is incorporated in hydrogels as described herein. 40 normoglycemic (wild type (WT)) female mice of 7 weeks old were wounded with an 8 mm circular biopsy needle to create one full-thickness skin wound on the back skin of each mouse. A group of 10 mice was treated with Tegaderm^{™}, a group of 10 mice was treated with 5% Glycyrrhetinic acid (5% GA) and a group of 10 mice was treated with 10% Glycyrrhetinic acid (10% GA). Treatment continued up to 14 days after wounding. Figure 21 shows that a hydrogel containing 5% GA is more optimal than a hydrogel consisting of 15% GA for wound healing in mice.

### Materials and methods

Glycyrrhizic acid monoammonium salt (Gly) was purchased from Sigma Aldrich and used without any prior purification. EG 56 (bis-methoxy PEG₁₃-PPG₄₃₈-PPG₁₁₀) was obtained from Polymer expert. Sterilized water was used.

All samples were prepared in a laminar flow decreasing the risk on bacterial contamination. 50 mg of Gly was dissolved in 1000 mg of sterilized water and these samples were then heated to dissolve the Gly to obtain the Gly (5 m%) sample. For the control sample 0.5 g of EG56 was added to the Gly (5%) sample.

The rheology of the gels was tested on a MCR 501 Rheometer. The sample was a gel and therefore heated to 60°C to induce degelation. The first measurement included a temperature sweep were both G' (elastic modulus) and G" (loss modulus) are plotted in function of the temperature, strain 1% and frequency 10 rad/s. The next measurements were done at 37°C and 20°C when the sample was equilibrated. The strain sweep (10 rad/s) determined if the measurement was done in the linear domain. The following frequency sweep was done according to the obtained values in the strain sweep. The stress sweep shows the viscosity related to the shear stress to evaluate whether the hydrogels qualify as injectables.

Six-weeks old female C57/BI6J mice were obtained from Janvier and wounded at week 7 of age. Specifically, mice were anaesthetized with isoflurane and subcutaneously injected with the analgesic Vetergesics and the back skin was shaved with clippers. Mice were wounded with a disposable 8 mm punch biopsy needle (Stiefel). A single full-thickness skin wound was inflicted per mouse. Newly-inflicted wounds were treated with 0,5 ml of glycyrrhizin hydrogel, EG56/5% Gly hydrogel or left untreated. At day 2, 4, 6, 8 and 10 post-wounding, mice were again treated with 0,5 g of glycyrrhizin hydrogel, EG56/5% Gly hydrogel or left untreated. Wound healing was measured every other day until wound closure by means of electronic calipers.

### References

(1) Dreifke, M. B.; Jayasuriya, A. A.; Jayasuriya, A. C. Mater. Sci. Eng. C 2015, 48, 651.
(2) Eccleston, G.; Stevens, H. N.; Matthews, K. H.; Boateng, J. J. Pharm. Sci. 2008, 97 (8), 2892.
(3) Agrawal, P.; Soni, S.; Mittal, G.; Bhatnagar, A. Int. J. Low. Extrem. Wounds 2014, 13 (3), 180.
(4) Sood, A.; Granick, M. S.; Tomaselli, N. L. Adv. Wound Care 2014, 3 (8), 511.
(5) Mustoe, T. American Journal of Surgery. 2004,*.*
(6) Morin, R. J.; Tomaselli, N. L. Clin. Plast. Surg. 2007, 34 (4), 643.
(7) Schäfer, M.; Werner, S. Nat. Rev. Mol. Cell Biol. 2008, 9 (8), 628.
(8) Langer, A.; Rogowski, W. BMC Heal. Serv Res 2009, 9, 115.
(9) McIntosh, C.; Newton, V. Low. Extrem. Wounds 2008, 191.
(10) Hoffman, A. S. Adv. Drug Deliv. Rev. 2002, 64, 3.
(11) Moody, A. Br. J. Community Nurs. 2006, 11 (June), S12.
(12) Kamoun, E. A.; Kenawy, E. R. S.; Chen, X. J. Adv. Res. 2017, 8 (3), 217.
(13) Mercuri, L. Pharmaceutical composition based on glycyrrhizin and EG56 polymer for the prepartion of anti-inflamatory products/WO2011111084, 2011.
(14) Hoever, G.; Baltina, L.; Michaelis, M.; Kondratenko, R.; Baltina, L.; Tolstikov, G. A.; Doerr, H. W.; Cinatl, J. J. Med. Chem. 2005, 48, 1256.
(15) Keda, T. I.; Okomizo, K. Y.; Kawa, M. O.; Suchihashi, R. T.; Injo, J. K. Biol. Pharm. Bull 2005, 28 (September), 1779.
(16) Raffetseder, J.; Hauner, M.; Wolkerstorfer, A.; Ernst, W. **2009,** *482,* 473.
(17) Kondratenko, R. M.; Baltina, L. A.; Baltina. Jr., L. A.; Plyasunova, O. A.; Pokrovskii, A. G.; Tolstikov, G. A. Pharm. Chem. J. 2009, 43 (7), 383.
(18) Kao, T. C.; Shyu, M. H.; Yen, G. C. J. Agric. Food Chem. 2010, 58 (15), 8623.
(19) Yoshida, T.; Yoshida, S.; Kobayashi, M.; Herndon, D. N.; Suzuki, F. J. Leukoc. Biol. 2010**,** *87* (1), 35.
(20) Chen, X.; Fang, D.; Li, L.; Chen, L.; Li, Q.; Gong, F. Immunol. Res. 2017**.**
(21) Xiong, X.; Gu, L.; Wang, Y.; Luo, Y.; Zhang, H.; Lee, J.; Krams, S.; Zhu, S.; Zhao, H. J. Neuroinflammation 2016, 13 (241), 115.
(22) Mollica, L.; Marchis, F. De; Spitaleri, A.; Dallacosta, C.; Pennacchini, D.; Zamai, M.; Agresti, A.; Trisciuoglio, L.; Musco, G.; Bianchi, M. E. Chem. Biol. 2007, No. April, 431.
(23) Yang, P.-S.; Kim, D.-H.; Lee, Y. J.; Lee, S.-E.; Kang, W. J.; Chang, H.-J.; Shin, J.-S. New Engl. J. Med. 2014, 15 (18), 111.
(24) Yamaguchi, H.; Kidachi, Y.; Kamiie, K.; Noshita, T.; Umetsu, H. Bioinformation 2012, 8 (23), 1147.
(25) Ekanayaka, S. A.; McClellan, S. A.; Barrett, R. P.; Kharotia, S.; Hazlett, L. D. Investig. Opthalmology Vis. Sci. 2016, 57 (13), 5799.
(26) Bag, B. G.; Majumdar, R. RSC Adv. 2012, 2 (23), 8623.
(27) Majumdar, R.; Bag, B. G.; Maity, N. Int. Nano Lett. 2013, 3 (1), 53.
(28) Dash, S. S.; Majumdar, R.; Sikder, A. K.; Bag, B. G.; Patra, B. K. Appl. Nanosci. 2013, 4 (4), 485.
(29) Wu, J.; Lu, J.; Hu, J.; Gao, Y.; Ma, Q.; Ju, Y. RSC Adv. 2013, 3 (47), 24906.
(30) Wan, Z.; Sun, Y.; Ma, L.; Guo, J.; Wang, J.; Yin, S.; Yang, X. Food Funct. 2017, 8 (1), 75.
(31) Bras, W.; Derbyshire, G. E.; Ryan, A. J.; Mant, G. R.; Manning, P.; Cameron, R. E.; Mormann, W. J. Phys. IV JP 1993, 3 (8).
(32) Baltina, L. a. Curr. Med. Chem. 2003, 10 (2), 155.

## Claims

1. A hydrogel for use in assisting wound healing, essentially consisting of 3,5%-25% by weight of glycyrrhizic acid and water.

2. A hydrogel for use according to claim 1, for use to treat wound healing wherein said wound comprises a decubitus ulcer, a chronic ulcer and a diabetic ulcer.

3. Hydrogel for use according to claim 1 or 2, consisting of 3.5%-25% by weight of glycyrrhizic acid and water.

4. A topical plaster comprising a hydrogel essentially consisting of 3,5%-25% by weight of glycyrrhizic acid and water.

5. A topical plaster according to claim 4, for use in assisting wound healing.

6. A topical plaster according to claim 4 or for use according to claim 5, comprising a hydrogel consisting of 3,5%-25% by weight of glycyrrhizic acid and water.

## Patentansprüche

1. Hydrogel zur Verwendung beim Unterstützen der Wundheilung, im Wesentlichen aus 3,5 bis 25 Gew.-% Glycyrrhizinsäure und Wasser bestehend.

2. Hydrogel zur Verwendung nach Anspruch 1, zur Verwendung bei der Behandlung von Wundheilung, wobei die Wunde Druckgeschwüre, chronische Geschwüre und diabetische Geschwüre umfasst.

3. Hydrogel zur Verwendung nach Anspruch 1 oder 2, aus 3,5 bis 25 Gew.-% Glycyrrhizinsäure und Wasser bestehend.

4. Topisches Pflaster, ein Hydrogel umfassend, das im Wesentlichen aus 3,5 bis 25 Gew.-% Glycyrrhizinsäure und Wasser besteht.

5. Topisches Pflaster nach Anspruch 4 zur Verwendung beim Unterstützen der Wundheilung.

6. Topisches Pflaster nach Anspruch 4 oder zur Verwendung nach Anspruch 5, ein Hydrogel umfassend, das aus 3,5 bis 25 Gew.-% Glycyrrhizinsäure und Wasser besteht.

## Revendications

1. Hydrogel destiné à faciliter la cicatrisation des plaies, constitué essentiellement de 3,5 % à 25 % en poids d'acide glycyrrhizique et d'eau.

2. Hydrogel destiné à être utilisé selon la revendication 1, destiné à traiter la cicatrisation des plaies, dans lequel ladite plaie comprend un ulcère de décubitus, un ulcère chronique et un ulcère diabétique.

3. Hydrogel destiné à être utilisé selon la revendication 1 ou 2, composé de 3,5 % à 25 % en poids d'acide glycyrrhizique et d'eau.

4. Emplâtre topique comprenant un hydrogel constitué essentiellement de 3,5 % à 25 % en poids d'acide glycyrrhizique et d'eau.

5. Emplâtre topique selon la revendication 4, destiné à faciliter la cicatrisation des plaies.

6. Emplâtre topique selon la revendication 4 ou destin à être utilisé selon la revendication 5, comprenant un hydrogel composé de 3,5 % à 25 % en poids d'acide glycyrrhizique et d'eau.
